# EUROPÄISCHE PATENTSCHRIFT

(11) **EP 2 303 879 B1**
(45) Veröffentlichungstag und Bekanntmachung des Hinweises auf die Patenterteilung: **05.09.2012**
(21) Anmeldenummer: 09777108.3
(22) Anmeldetag: 10.07.2009
(51) Int. Cl.: C07D 471/04, A61K 31/437, A61P 3/10, A61P 3/04

(54) **7-AZAINDOLDERIVATE**
7-AZAINDOLE DERIVATIVES
DÉRIVÉS DE 7-AZAINDOLE

(30) Priorität: 18.08.2008 DE 102008038221
(43) Veröffentlichungstag der Anmeldung: 06.04.2011
(73) Patentinhaber: Merck Patent GmbH, 64293 Darmstadt (DE)
(72) Erfinder: KLEIN, Markus, 64291 Darmstadt (DE)
(86) Internationale Anmeldenummer: PCT/EP2009/005024
(87) Internationale Veröffentlichungsnummer: WO 2010/020308

(56) Entgegenhaltungen:
- WO-A-2004/078756

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Proteinkinasen, eine Rolle spielt, ferner pharmazeutische Zusammensetzungen, die diese Verbindungen enthalten, sowie die Verwendung der Verbindungen zur Behandlung kinase-bedingter Krankheiten.
Beispiele von Kinasen sind insbesondere die Tyrosinkinasen und/oder Serin/Threonin-Kinasen.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Rolle der Rezeptortyrosinkinase Met bei der menschlichen Onkogenese, sowie die Möglichkeit der Inhibierung der HGF(hepatocycte growth factor)-abhängigen Met-Aktivierung wird von S. Berthou et al. in Oncogene, Vol. 23, Nr. 31, Seiten 5387-5393 (2004) beschrieben. Der dort beschriebene Inhibitor SU11274, eine Pyrrol-Indolin-Verbindung, ist potentiell zur Krebsbekämpfung geeignet.
Ein anderer Met-Kinase-Inhibitor zur Krebstherapie ist von J.G. Christensen et al. in Cancer Res. 2003, 63(21), 7345-55 beschrieben.
Von einem weiterem Tyrosinkinase-Inhibitor zur Krebsbekämpfung berichten H. Hov et al. in Clinical Cancer Research Vol. 10, 6686-6694 (2004). Die Verbindung PHA-665752, ein Indolderivat, ist gegen den HGF-Rezeptor c-Met gerichtet. Weiter wird dort berichtet, daß HGF und Met erheblich zum malignen Prozess verschiedener Krebsformen, wie z.B. multipler Myeloma, betragen.

Zu anderen bevorzugten Kinasen gehört die zellvolumenregulierte humane Kinase h-sgk (human serum and glucocorticoid dependent kinase oder SGK).
Die SGK mit den Isoformen SGK-1, SGK-2 und SGK-3 sind eine Serin/Threonin-Proteinkinase Familie (WO 02/17893).

Die Synthese von kleinen Verbindungen, die die Signaltransduktion der Tyrosinkinasen und/oder Serin/Threonin-Kinasen, insbesondere der Metund/oder SGK spezifisch hemmen, regulieren und/oder modulieren, ist daher wünschenswert und ein Ziel der vorliegenden Erfindung.

Die erfindungsgemäßen Verbindungen sind vorzugsweise selektive Inhibitoren der SGK-1. Ferner können sie Inhibitoren der SGK-2 und/oder SGK-3 sein. Im einzelnen betrifft die vorliegende Erfindung Verbindungen, die die Signaltransduktion der SGK hemmen, regulieren und/oder modulieren, Zusammensetzungen, die diese Verbindungen enthalten, sowie Verfahren zu ihrer Verwendung zur Behandlung von SGK-bedingten Krankheiten und Leiden wie Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).
Die erfindungsgemäßen Verbindungen können auch das Wachstum von Tumorzellen und Tumormetastasen hemmen und sind deshalb für die Tumortherapie geeignet.
Die erfindungsgemäßen Verbindungen finden auch Verwendung bei der Behandlung von peptischen Ulcera, insbesondere bei Formen, die durch Stress ausgelöst werden.
Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, Prothrombin-Komplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden. Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden. Darüberhinaus wirken die erfindungsgemäßen Verbindungen der Zellalterung und Stress entgegen und steigern somit die Lebenserwartung und die Fitness im Alter.
Die erfindungsgemäßen Verbindungen finden ferner Verwendung bei der Behandlung von Tinitus.

Im einzelnen betrifft die vorliegende Erfindung weiterhin Verbindungen der Formel I, die die Signaltransduktion der Met-Kinase hemmen, regulieren und/oder modulieren, Zusammensetzungen, die diese Verbindungen enthalten, ihre Verwendung zur Herstellung eines Arzneimittels zur Behandlung von MetKinasebedingten Krankheiten und Leiden wie Angiogenese, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Feste Tumore, insbesondere schnell wachsende Tumore, können mit Met-Kinasehemmern behandelt werden. Zu diesen festen Tumoren zählen die Monozytenleukämie, Hirn-, Urogenital-, Lymphsystem-, Magen-, Kehlkopfund Lungenkarzinom, darunter Lungenadenokarzinom und kleinzelliges Lungenkarzinom.

Die vorliegende Erfindung richtet sich auch auf Verfahren zur Regulation, Modulation oder Hemmung der Met-Kinase zur Vorbeugung und/oder Behandlung von Erkrankungen im Zusammenhang mit unregulierter oder gestörter Met-Kinase-Aktivität. Insbesondere lassen sich die Verbindungen der Formel I auch bei der Behandlung gewisser Krebsformen einsetzen. Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I zur Isolierung und zur Untersuchung der Aktivität oder Expression von Met-Kinase verwendet werden. Außerdem eigenen sie sich insbesondere zur Verwendung in diagnostischen Verfahren zu Erkrankungen im Zusammenhang mit unregulierter oder gestörter Met-Kinase-Aktivität.

Die Identifikation von kleinen Verbindungen, die die Signaltransduktion der SGK- und/oder Met-Kinase spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.
Insbesondere zeigen sie inhibierende Eigenschaften der SGK und/oder Met-Kinase.

Die erfindungsgemäßen Verbindungen zeigen weiterhin Aktivität auf andere Kinasen wie Aurora-B, MAPK2, MSK1, PRK2, DYRK1, CHK2, GSK3-beta, PKB (AKT), ROCKII oder S6K1, limk1, TGF-beta, MAPK8, PLK1, PDK1, MKK1, SAPK3, SAPK4, und andere.

Heterocyclische Verbindungen mit inhibitorischer Wirkung auf GSK3-beta und den damit verbundenen Krankheitsbildern sind z.B. in der WO 2008/078196 beschrieben.
Die erfindungsgemäßen Verbindungen können daher zur Behandlung neurodegenerativen Erkrankungen, wie z.B. Parkinson, Tauopathien, wie z.B. Morbus Alzheimer, corticobasale Degeneration, Morbus Pick, Morbus Wilson, Morbus Huntington, weiterhin vaskuläre Dementia, akuter Schlaganfall, periphere Neuropathien, Retinipathie oder Glaucom, ferner manisch depressive Erkrankungen. Durch die Inhibierung von GSK3-beta können die Verbindungen auch zur Behandlung von Krebs und Tumorerkrankungen verwendet werden.

Die erfindungsgemäßen Verbindungen können darüber hinaus zur Behandlung von Autoimmunerkrankungen, inflammatorischen und proliferativen Erkrankungen, AIDS, Asthma, Rhinitis und Morbus Crohn verwendet werden.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemolumineszenz nachweisbar (Ross et al., Biochem. J., 2002, 366, 977-981).

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

### STAND DER TECHNIK

Andere 1 H-Pyrrolo(2,3-b)pyridine sind als Proteinkinase-Inhibitoren in WO 2005/085244 A1 und in EP 1749829 beschrieben.
Andere Azaindolderivate sind als Proteinkinase-Inhibitoren in WO 2006/004984 beschrieben, die zur Behandlung von Autoimmunerkrankungen, inflammatorischen und proliferativen Erkrankungen, AIDS, Asthma, Rhinitis und Morbus Crohn verwendet werden können.
Wiederum andere Pyrrolopyridine sind als Proteinkinase-Inhibitoren in WO 2004/078756 A2 beschrieben.
Indole und andere heterocyclische Derivate sind als Kinase-Inhibitoren in US 2005/250829 offenbart.
Wiederum andere Azaindole sind als Proteinkinase-Inhibitoren in WO 2005/1030050 A2 beschrieben.
Andere heterocyclische Indolderivate sind als Kinase-Inhibitoren in WO 2005/123672 A2 offenbart.
Andere heterocyclische Oxadiazolderivate kennt man aus WO 2002/72549 A1.
Heteroarylverbindungen zur Behandlung von Krebs sind in WO 2003/040402 beschrieben.

In der WO 00/62781 ist die Verwendung von Arzneimitteln enthaltend Hemmstoffe der zellvolumenregulierten humanen Kinase H-SGK beschrieben. Heterocyclische Indazolderivate zur Behandlung von Diabetes und/oder Krebserkrankungen kennt man aus WO 2006/044860 und WO 2005056550. In US 2005090529 sind Indazolderivate zur Behandlung von diabetischer Retinopathie offenbart.
Indazolderivate zur Behandlung von Tumoren sind in WO 2005000813 offenbart, solche zur Behandlung von cardiovasculärer Erkrankungen in WO 2004060318.
Andere heterocyclische Verbindungen zur Behandlung von Tumoren kennt man aus W02004052280.
Ferner sind andere Heterocyclen zur Behandlung psychotischer Erkrankungen in EP 328200 offenbart.
Indazolderivate sind als Proteinkinase-Inhibitoren in der WO 03/064397 beschrieben.
In Bioorganic & Medicinal Chemistry Letters 13 (2003) 3059-3062 beschreibt J. Witherington et al. die Herstellung von Indazolderivaten.
Indazolderivate sind als Kinaseinhibitoren in WO 2003097610 beschrieben. Indazolderivate sind als GSK-3-Inhibitoren in WO 2003051847 offenbart. Triazolo-pyridazinderivate sind als Met-Kinase-Inhibitoren beschrieben in WO 2007/064797, WO 2007/075567, WO 2007/138472, WO 2008/008539, WO 2008/051805.

Die Verwendung von Kinase-Inhibitoren in der antiinfektiösen Therapie ist von C.Doerig in Cell. Mol. Biol. Lett. Vol.8, No. 2A, 2003, 524-525 beschrieben. Die Verwendung von Kinase-Inhibitoren bei Fettsucht ist von N.Perrotti in J. Biol. Chem. 2001, März 23; 276(12):9406-9412 beschrieben.

In nachstehenden Literaturstellen wird die Verwendung von SGK-Hemmern bei der Behandlung von Krankheiten nahegelegt und/oder beschrieben:
1: Chung EJ, Sung YK, Farooq M, Kim Y, Im S, Tak WY, Hwang YJ, Kim YI, Han HS, Kim JC, Kim MK. Gene expression profile analysis in human hepatocellular carcinoma by cDNA microarray. Mol Cells. 2002;14:382-7.
2: Brickley DR, Mikosz CA, Hagan CR, Conzen SD. Ubiquitin modification of serum and glucocorticoid-induced protein kinase-1 (SGK-1). J Biol Chem. 2002;277:43064-70.
3: Fillon S, Klingel K, Wamtges S, Sauter M, Gabrysch S, Pestel S, Tanneur V, Waldegger S, Zipfel A, Viebahn R, Haussinger D, Broer S, Kandolf R, Lang F. Expression of the serine/threonine kinase hSGK1 in chronic viral hepatitis. Cell Physiol Biochem. 2002;12:47-54.
4: Brunet A, Park J, Tran H, Hu LS, Hemmings BA, Greenberg ME. Protein kinase SGK mediates survival signals by phosphorylating the forkhead transcription factor FKHRL1 (FOXO3a). Mol Cell Biol 2001;21:952-65
5: Mikosz CA, Brickley DR, Sharkey MS, Moran TW, Conzen SD. Glucocorticoid receptor-mediated protection from apoptosis is associated with induction of the serine/threonine survival kinase gene, sgk-1. J Biol Chem. 2001; 276:16649-54.
6: Zuo Z, Urban G, Scammell JG, Dean NM, McLean TK, Aragon I, Honkanen RE. Ser/Thr protein phosphatase type 5 (PP5) is a negative regulator of glucocorticoid receptor-mediated growth arrest. Biochemistry. 1999;38:8849-57.
7: Buse P, Tran SH, Luther E, Phu PT, Aponte GW, Firestone GL. Cell cycle and hormonal control of nuclear-cytoplasmic localization of the serum- and glucocorticoid-inducible protein kinase, Sgk, in mammary tumor cells. A novel convergence point of anti-proliferative and proliferative cell signalling pathways. J Biol Chem. 1999;274:7253-63.
8: M. Hertweck, C. Göbel, R. Baumeister: C.elegans SGK-1 is the critical component in the Akt/PKB Kinase complex to control stress response and life span. Developmental Cell, Vol. 6, 577-588, April, 2004.

### ZUSAMMENFASSUNG DER ERFINDUNG

Die Erfindung betrifft Verbindungen der Formel I 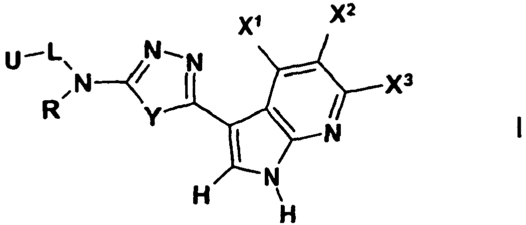 worin
- L: fehlt, CR⁷R⁸, CR⁷R⁸CR⁹R¹⁰, CR⁷R^{B}C(OR⁹)R¹⁰, NR⁷, O, NR⁶CR⁷R⁸, CR⁷R⁸NR⁹, OCR⁷R⁸, OCR⁷R⁸CR⁹R¹⁰, CR⁷R⁸O, CR⁷R⁸CR⁹R¹⁰O, NR⁶CR⁷R⁸CR⁹R¹⁰, CR⁷R⁸SO₂, NR⁷CONR⁸, NR⁷CONR⁸CR⁹R¹⁰, COCR⁷R⁸, CONR⁷, CONR⁷CR⁸R⁹, CONHNH, NR⁷CR⁸R⁹CONR¹⁰, NR⁷CO oder NR⁷COCR⁸R⁹,
- U: H, A, Ar oder Het,
- Y: O, NH, NNH₂ oder N-[C(R⁷)₂]ₙAr,
- R H oder: R¹¹,
- X¹, X², X³: jeweils unabhängig voneinander H, A, Hal, OH, OA, -[C(R⁷)₂]ₙAr, -[C(R⁷)₂]ₙHet, OAr, OHet, SH, SA, SAr, SHet, NH₂, NHA, NAA', NHAr, N(Ar)₂, NHHet, N(Het)₂, NAAr, NAHet, SOA, SOAr, SOHet, SO₂A, SO₂Ar, SO₂Het, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr, COHet, SO₃H, SO₂NH₂, SO₂NHAr, SO₂N(Ar)₂, SO₂NHHet oder SO₂N(Het)₂,
- R⁶, R⁷, R⁸, R⁹, R¹⁰ R¹¹: jeweils unabhängig voneinander H oder A, Alkyl mit 1-6 C-Atomen, worin 1-5 H-Atome durch F ersetzt sein können,
- A, A': jeweils unabhängig voneinander unsubstituiertes oder ein-, zwei- oder dreifach durch =S, =NR⁷ und/oder =O (Carbonylsauerstoff) substituiertes Alkyl mit 1-10 C-Atomen, worin eine, zwei oder drei CH₂-Gruppen durch O, S, SO, SO₂, NH, NR¹¹ und/oder durch -CH=CH-Gruppen und/oder auch 1-7 H-Atome durch F und/oder Cl ersetzt sein können, oder cyclisches Alkyl mit 3-7 C-Atomen,
- Ar: unsubstituiertes oder ein-, zwei-, drei-oder vierfach durch A, Hal, OH, OA, Ar', OAr', Het, OHet, SH, SA, SAr', SHet, NH₂, NHA, NAA', NHAr', N(Ar')₂, NHHet, N(Het)₂, NAAr', NAHet, SOA, SOAr', SOHet, SO₂A, SO₂Ar', SO₂Het, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr'. COHet, SO₃H, SO₂NH₂, SO₂NHAr', SO₂N(Ar')₂, SO₂NHHet und/oder SO₂N(Het)₂ substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der ein-, zwei- oder dreifach durch A, Hal, OH, OA, Ar, OAr, Het', OHet', SH, SA, SAr', SHet', NH₂, NHA, NAA', NHAr', N(Ar')₂, NHHet', N(Het')₂, NAAr', NAHet', SOA, SOAr', SOHet', SO₂A, SO₂Ar', SO₂Het', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr'. COHet', SO₃H, SO₂NH₂, SO₂NHAr', SO₂N(Ar')₂, SO₂NHHet' oder SO₂N(Het')₂. =S, =NR⁷ und/oder =O (Carbonylsauerstoff) substituiert sein kann,
- Ar': unsubstituiertes oder ein-, zwei-, drei-oder vierfach durch A, Hal, OH, OA, OPhenyl, SH, SA, NH₂, NHA, NAA', NHPhenyl, SOA, SOPhenyl, SO₂A, SO₂Phenyl, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COPhenyl, SO₃H, SO₂NH₂, SO₂NHPhenyl und/oder SO₂N(Phenyl)₂ substituiertes Phenyl,
- Het': einen ein- oder zweikernigen gesättigten, ungesättigten oder aromatischen Heterocyclus mit 1 bis 4 N-, O- und/oder S-Atomen, der ein-, zwei- oder dreifach durch A, Hal, OH, OA, NH₂, NHA, NAA', SO_{A}, SOAr', SO₂A, SO₂Ar', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', SO₃H, SO₂NH₂, SO₂NHAr', SO₂N(Ar')₂, =S, =NR⁷ und/oder =O (Carbonylsauerstoff) substituiert sein kann,
- Hal: F, Cl, Br oder I,
- n: 0, 1 oder 2,
sowie ihre pharmazeutisch verwendbaren Salze, Solvate und Stereoisomere, einschließlich deren Mischungen in allen Verhältnissen.

Gegenstand der Erfindung sind die Verbindungen der Formel I und ihre Salze sowie ein Verfahren zur Herstellung von Verbindungen der Formel I sowie ihrer pharmazeutisch verwendbaren Salze und Stereoisomere, dadurch gekennzeichnet, daß man
a) eine Verbindung der Formel II 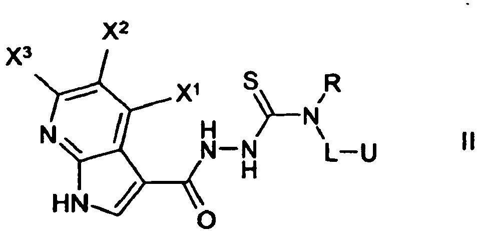 worin X¹, X², X³, R, L und U die in Anspruch 1 angegebenen Bedeutungen haben,
   cyclisiert,
   oder
b) eine Verbindung der Formel **III** 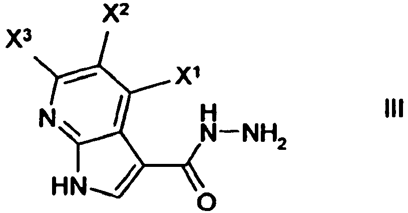 worin X¹, X², X³ die in Anspruch 1 angegebenen Bedeutungen haben,
   mit einem Cyanhalogenid umsetzt,
   oder
c) eine Verbindung der Formel I, worin Y Sauerstoff bedeutet, mit einem Hydrazinderivat oder mit H₂N-[C(R⁷)₂]ₙAr zu einer Verbindung der Formel I umwandeln, worin Y NNH₂ oder N-(C(R⁷)₂]ₙAr bedeutet,
   und/oder eine Base oder Säure der Formel I in eines ihrer Salze umwandelt.

Unter den Verbindungen der Formel I versteht man auch die Hydrate und Solvate dieser Verbindungen. Gegenstand der Erfindung sind auch die Stereoisomeren (E, Z-Isomeren) sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens,
einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen der Formel I, z.B. Gemische zweier Diastereomerer oder Enantiomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen, insbesondere liegen die erfindungsgemäßen Verbindungen als Racemat vor.

Für alle Reste, die mehrfach auftreten, gilt, daß deren Bedeutungen unabhängig voneinander sind.
Vor- und nachstehend haben die Reste bzw. Parameter L, U, R, Y, X¹, X² und X³ die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3-oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
L bedeutet vorzugsweise "fehlt" oder CR⁷R⁸, wie CH₂.
R bedeutet vorzugsweise H.

Ar bedeutet z.B. Phenyl, o-, m- oder p-Tolyl, o-, m- oder p-Ethylphenyl, o-, m-oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m-oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m-oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Cyanphenyl, o-, m- oder p-Ureidophenyl, o-, m- oder p-Formylphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino-oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise unsubstituiertes oder ein-, zwei-, drei- oder vierfach durch A, Hal, OH und/oder OA substituiertes Phenyl, wie z.B. o-, m-oder p-Methoxyphenyl, o-, m- oder p-Hydroxyphenyl, o-, m- oder p-Fluorphenyl, o-, m- oder p-Chlorphenyl, 2,3-, 2,4-, 2,5-, 2,6-, 3,4- oder 3,5-Dichlorphenyl, 2,3-, 2,4-, 2,5-, 2,6-, 3,4-, 3,5-Difluorphenyl oder 3-Chlor-4-fluorphenyl.

Ar' bedeutet vorzugsweise Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m-oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Cyanphenyl, o-, m- oder p-Ureidophenyl, o-, m- oder p-Formylphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino-oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Het bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxomethylendioxy)-phenyl oder auch 3,4-Dihydro-2H-1,5-benzodioxepin-6- oder - 7-yl, ferner bevorzugt 2,3-Dihydrobenzofuranyl oder 2,3-Dihydro-2-oxo-furanyl.

Het bedeutet vorzugsweise einen einkernigen aromatischen Heterocyclus mit 1 bis 4 N-, O- und/oder S-Atomen.

Het bedeutet besonders bevorzugt 2- oder 3-Furyl, 2- oder 3-Thienyl, 1-, 2-oder 3-Pyrrolyl, 1-, 2, 4- oder 5-Imidazolyl, 1-, 3-, 4- oder 5-Pyrazolyl, 2-, 4-oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl oder Pyrazinyl.

Het bedeutet ganz besonders bevorzugt Pyrrolyl, 2-, 3- oder 4-Pyridyl, 2- oder 3-Furyl, 2- oder 3-Thienyl.

Het' bedeutet vorzugsweise einen einkemigen gesättigten, ungesättigten oder aromatischen Heterocyclus mit 1 bis 2 N- und/oder O-Atomen, der unsubstituiert oder ein-, zwei- oder dreifach durch A, Hal, OH und/oder OA substituiert sein kann.
In einer weiteren Ausführungsform bedeutet Het' besonders bevorzugt unsubstituiertes oder ein-, zwei- oder dreifach durch A, Hal, OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl.

R⁶, R⁷, R⁸, R⁹, R¹⁰ bedeuten vorzugsweise, jeweils unabhängig voneinder, H oder R¹¹, ganz besonders bevorzugt H oder Methyl.

X¹, X², X³ bedeuten vorzugsweise, jeweils unabhängig voneinander H, Hal oder -[C(R⁷)₂]ₙHet.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Ik ausgedrückt werden, die der Formel I entsprechen und worin die nicht näher bezeichneten Reste die bei der Formel I angegebene Bedeutung haben, worin jedoch
- in Ia L: fehlt oder CR⁷R⁸ bedeutet;
- in Ib R: H bedeutet;
- in Ic A: Alkyl mit 1-10 C-Atomen, worin 1-7 H-Atome durch F und/oder Cl ersetzt sein können, bedeutet;
- in Id Ar: unsubstituiertes oder ein-, zwei-, drei- oder vierfach durch A, Hal, OH und/oder OA substituiertes Phenyl bedeutet;
- in le Het: einen einkernigen aromatischen Heterocyclus mit 1 bis 4 N-, O- und/oder S-Atomen;
- in If Het: 2- oder 3-Furyl, 2- oder 3-Thienyl, 1-, 2- oder 3-Pyrrolyl, 1-, 2, 4- oder 5-imidazolyl, 1-, 3-, 4- oder 5-Pyrazolyl, 2-, 4-oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl oder Pyrazinyl, bedeutet;
- in Ig R¹, R⁸: jeweils unabhängig voneinander H oder R¹¹, bedeuten;
- in Ih R⁷, R⁸: jeweils unabhängig voneinander H oder CH₃, bedeuten;
- in Ii Het: Pyrrolyl, 2-, 3- oder 4-Pyridyl, 2- oder 3-Furyl oder 2- oder 3-Thienyl, bedeutet;
- in lj X¹, X²,: X³ jeweils unabhängig voneinander H, Hal oder -[C(R¹)₂]ₙHet, bedeuten;
- in Ik L: fehlt oder CR⁷R⁸,
- U: H, A, Ar oder Het,
- Y: O, NH, NNH₂ oder N-[C(R⁷)₂]ₙAr,
- R: H oder R¹¹,
- X¹, X², X³: jeweils unabhängig voneinander H, Hal oder -[C(R⁷)₂]ₙHet,
- R⁷, R³: jeweils unabhängig voneinander H oder R¹¹,
- R¹¹: Alkyl mit 1-6 C-Atomen, worin 1-5 H-Atome durch F ersetzt sein können,
- A: Alkyl mit 1-10 C-Atomen, worin 1-7 H-Atome durch F und/oder Cl ersetzt sein können,
- Ar: unsubstituiertes oder ein-, zwei-, drei- oder vierfach durch A, Hal, OH und/oder OA substituiertes Phenyl,
- Het: einen einkernigen aromatischen Heterocyclus mit 1 bis 4 N-, O- und/oder S-Atomen,
- Hal: F, Cl, Br oder I,
- n: 0, 1 oder 2, bedeuten;
sowie ihre pharmazeutisch verwendbaren Salze und Stereoisomere, einschließlich deren Mischungen in allen Verhältnissen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I, worin Y Sauerstoff bedeutet, können vorzugsweise erhalten werden, indem man Verbindungen der Formel II cyclisiert. Die Cyclisierung erfolgt vorzugsweise unter Zusatz eines Quecksilbersalzes in einem inerten Lösungsmittel.
Als Quecksilberslz ist Quecksilber (II)acetat besonders bevorzugt.
Die Reaktionszeit liegt je nach den angewendeten Bedingungen zwischen einigen Minuten und 14 Tagen, die Reaktionstemperatur zwischen etwa -30° und 140°, normalerweise zwischen 0° und 100°, insbesondere zwischen etwa 60° und etwa 90°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Methanol oder Ethanol.

Verbindungen der Formel I, worin
- Y: Sauerstoff,
- R: H,
- L: fehlt,
- U: H,
bedeuten,
können weiterhin bevorzugt erhalten werden, indem man Verbindungen der Formel III mit einem Cyanhalogenid, vorzugsweise BrCN, umsetzt.
Die Umsetzung erfolgt in einem inerten Lösungsmittel, wie oben angegeben, vorzugsweise in Wasser und/oder DMF.
Die Reaktionszeit liegt je nach den angewendeten Bedingungen zwischen einigen Minuten und 14 Tagen, die Reaktionstemperatur zwischen etwa -30° und 140°, normalerweise zwischen 0° und 100°, insbesondere zwischen etwa 15° und etwa 70°.

Die Umsetzung erfolgt in der Regel in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin kann günstig sein.

Verbindungen der Formel I, worin
Y NNH₂ oder N-[C(R⁷)₂]ₙAr bedeutet,
können weiterhin bevorzugt erhalten werden, indem man Verbindungen der Formel I, worin Y Sauerstoff bedeutet, mit einem Hydrazinderivat, vorzugsweise Hydrazin, Hydrazinhydrat oder beispielsweise mit Benzylamin umsetzt.
Die Umsetzung erfolgt in einem inerten Lösungsmittel, wie oben angegeben, vorzugsweise in Propanol.
Die Reaktionszeit liegt je nach den angewendeten Bedingungen zwischen einigen Minuten und 14 Tagen, die Reaktionstemperatur zwischen etwa 0° und 160°, normalerweise zwischen 20° und 140°, insbesondere zwischen etwa 80° und etwa 120°.

Die Spaltung eines Ethers erfolgt unter Methoden, wie sie dem Fachmann bekannt sind.
Eine Standardmethode zur Etherspaltung, z.B. eines Methylethers, ist die Verwendung von Bortribromid.
Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere
z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Walzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Salze und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearytalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie deren Salze lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per* se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Salze und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Salze und Stereoisomere, einschließlich deren Mischungen in allen Verhältnissen, und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von SGK-bedingten Krankheiten.

Gegenstand der Erfindung ist somit die Verwendung von Verbindungen nach Anspruch 1, sowie ihrer pharmazeutisch verwendbaren Salze und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt. Bevorzugt ist hierbei SGK.

Bevorzugt ist die Verwendung von Verbindungen gemäß Anspruch 1, sowie ihrer pharmazeutisch verwendbaren Salze und Stereoisomere, einschließlich deren Mischungen in allen Verhältnissen, zur Herstellung eines Arzneimittels zur Behandlung von Krankheiten, die durch Inhibierung der SGK durch die Verbindungen nach Anspruch 1 beeinflußt werden.

Die vorliegende Erfindung umfasst die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).
Die erfindungsgemäßen Verbindungen können auch das Wachstum von Krebs, Tumorzellen und Tumormetastasen hemmen und sind deshalb für die Tumortherapie geeignet.
Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, Prothrombin-Komplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden. Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden.

Bevorzugt ist die Verwendung von Verbindungen gemäß Anspruch 1, sowie ihrer pharmazeutisch verwendbaren Salze und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress.

Bei Diabetes handelt es sich vorzugsweise um Diabetes mellitus, diabetische Nephropathie, diabetische Neuropathie, diabetische Angiopathie und Mikroangiopathie.

Bei Herzkreislauferkrankungen handelt es sich vorzugsweise um kardiale Fibrosen nach Myokardinfarkt, Herzhypertrophie, Herzinsuffizienz und Arteriosklerose.

Bei Nierenerkrankungen handelt es sich vorzugsweise um Glomerulosklerose, Nephrosklerose, Nephritis, Nephropathie und Störung der Elektrolytausscheidung.

Bei Fibrosen und entzündlichen Prozessen handelt es sich vorzugsweise um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer.

Zu den tyrosinkinasebedingten Krankheiten zählen auch die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).
Die vorliegende Erfindung umfasst weiterhin die Verwendung der Verbindungen der Formel I und/oder ihre physiologisch unbedenklichen Salze zur Herstellung eines Arzneimittels zur Behandlung oder Vorbeugung von Krebs. Bevorzugte Karzinome für die Behandlung stammen aus der Gruppe Himkarzinom, Urogenitaltraktkarzinom, Karzinom des lymphatischen Systems, Magenkärzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.
Ebensfalls umfasst ist die Verwendung der erfindungsgemäßen Verbindungen nach Anspruch 1 und/oder ihre physiologisch unbedenklichen Salze zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung der Verbindungen der Formel I und/oder ihre physiologisch unbedenklichen Salze zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.
Die vorliegende Erfindung umfasst auch die Verwendung der Verbindungen der Formel und/oder ihre physiologisch unbedenklichen Salze und Solvate zur Herstellung eines Arzneimittels zur Behandlung oder Vorbeugung von Retina-Vaskularisierung.

Die Verwendung zur Herstellung eines Arzneimittels zur Behandlung oder Vorbeugung von Augenkrankheiten wie diabetischer Retinopathie und altersbedingter Makula-Degeneration sind ebenfalls ein Bestandteil der Erfindung. Die Verwendung zur Herstellung eines Arzneimittels zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.
Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I können an Patienten zur Behandlung von Krebs, insbesondere schnell wachsenden Tumoren, verabreicht werden.

Gegenstand der Erfindung ist auch die Verwendung von Verbindungen der Formel I, sowie ihrer pharmazeutisch verwendbaren Salze und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt ist hierbei die Met-Kinase.

Bevorzugt ist die Verwendung von Verbindungen der Formel I, sowie ihrer pharmazeutisch verwendbaren Salze und Stereoisomere, einschließlich deren Mischungen in allen Verhältnissen,
zur Herstellung eines Arzneimittels zur Behandlung von Krankheiten, die durch Inhibierung der Tyrosinkinasen durch die Verbindungen nach Anspruch 1 beeinflußt werden.

Besonders bevorzugt ist die Verwendung zur Herstellung eines Arzneimittels zur Behandlung von Krankheiten, die durch Inhibierung von Met-Kinase durch die Verbindungen nach Anspruch 1 beeinflußt werden.
Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren der Lunge, des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineopfastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyhamstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-αvβ3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und Invivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthe |
| | Tetraplatin | BBR-3464 (Hoffmann-La Roche) |
| | Ormiplatin | |
| | Iproplatin | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyhamstoff |
| | 6-Thioguanin | Decitabin (SuperGen) |
| | Cytarabin | Clofarabin (Bioenvision) |
| | 2-Fluordesoxycytidin | Irofulven (MGI Pharma) |
| | Methotrexat | DMDC (Hoffmann-La Roche |
| | Idatrexate | Ethinylcytidin (Taiho ) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour- |
| | 7-Ethyl-10- | Ipsen) |
| | hydroxycamptothecin | TAS-103 (Taiho) |
| | Topotecan | Elsamitrucin (Spectrum) |
| | Dexrazoxanet (TopoTarget) | J-107088 (Merck & Co) |
| | Pixantron (Novuspharma) | BNP-1350 (BioNumerik) |
| | Rebeccamycin-Analogon (Exelixis) | CKD-602 (Chong Kun Dang |
| | | KW-2170 (Kyowa Hakko) |
| | BBR-3576 (Novuspharma) | |
| | | |
| Antitumor-Antibiotik | Dactinomycin (Actinomycin I | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubici | GPX-100 (Gem Pharmaceuticals) |
| | Mitoxantron (Novantron) | |
| | | |
| Antimitotische Mitte | Paclitaxel | SB 408075 (GlaxoSmithKlin |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCI) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B (PharmaMar) |
| | RPR 109881A (Aventis) | |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient |
| | Vinflunin (Fabre) | NeuroPharma) |
| | Auristatin PE (Teikoku Hormone) | Azaepothilon B (BMS) |
| | | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (Protarga) | |
| | | |
| Aromatase-Inhibitoren | Aminoglutethimid | Exemestan |
| | Letrozol | Atamestan (BioMedicines) |
| | Anastrazol Formestan | YM-511 (Yamanouchi) |
| | | |
| Thymidylatsynthase Inhibitoren | Pemetrexed (Eli Lilly) | Nolatrexed (Eximias) |
| | ZD-9331 (BTG) | CoFactor^{™} (BioKeys) |
| | | |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltransferase Inhibitoren | Arglabin (NuOncology Labs: | Tipifamib (Johnson & Johnson) |
| | lonafamib (Schering-Plough | |
| | BAY-43-9006 (Bayer) | Perillylalkohol (DOR BioPharma) |
| | | |
| Pumpen-Inhibitoren | CBT-1 (CBA Pharma) | Zosuquidar-Trihydrochlorid (Eli Lilly) |
| | Tariquidar (Xenova) | |
| | MS-209 (Schering AG) | Biricodar-Dicitrat (Vertex) |
| | | |
| Histonacetyltransferase-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidredul se-Inhibitoren | Neovastat (Aetema Laboratories) | CMT -3 (CollaGenex) |
| | | BMS-275291 (Celltech) |
| | Marimastat (British Biotech) | Tezacitabin (Aventis) |
| | Galliummaltolat (Titan) | Didox (Molecules for Health |
| | Triapin (Vion) | |
| | | |
| TNF-alpha-Agonisten / Antagonisten | Virulizin (Lorus Therapeutics | Revimid (Celgene) |
| | CDC-394 (Celgene) | |
| | | |
| Endothelin-A-Rezeptor-Antagonisten | Atrasentan (Abbot) | YM-598 (Yamanouchi) |
| | ZD-4054 (AstraZeneca) | |
| | | |
| Retinsäurerezeptor Agonisten | Fenretinid (Johnson & Johnson) | Alitretinoin (Ligand) |
| | LGD-1550 (Ligand) | |
| | | |
| Immunmodulatoren | Interferon | Dexosom-Therapie (Anosys |
| | Oncophage (Antigenics) | Pentrix (Australian Cancer |
| | GMK (Progenics) | Technology) |
| | Adenokarzinom-Impfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | !3-Alethin (Dovetail) |
| | | CLL-Thera (Vasogen) |
| | Melanom-Impfstoff (CTL Immuno) | |
| | p21-RAS-Impfstoff (GemVa: | |
| | | |
| Hormonelle und antihormonelle Mitt | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat Fluoxymesteron | Flutamid Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed) |
| | Toremofin | |
| | Dexamethason | Arzoxifen (Eli Lilly) |
| | | |
| Photodynamische Mittel | Talaporfin (Light Sciences) | Pd-Bacteriopheophorbid (Yeda) |
| | Theralux (Theratechnologie: | |
| | Motexafin-Gadolinium (Pharmacyclics) | Lutetium-Texaphyrin (Pharmacyclics) |
| | | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science | PKC412 (Novartis) |
| | Canertjnib (Pfizer) | Phenoxodiol O |
| | Squalamin (Genaera) | Trastuzumab (Genentech) |
| | SU5416 (Pharmacia) | C225 (ImClone) |
| | SU6668 (Pharmacia) | rhu-Mab (Genentech) |
| | ZD4190 (AstraZeneca) | MDX-H210 (Medarex) |
| | ZD6474 (AstraZeneca) | 2C4 (Genentech) |
| | Vatalanib (Novartis) | MDX-447 (Medarex) |
| | PKI166 (Novartis) | ABX-EGF (Abgenix) |
| | GW2016 (GlaxoSmithKline) | IMC-1C11 (ImClone) |
| | EKB-509 (Wyeth) | |
| | EKB-569 (Wyeth) | |
| Verschiedene Mitte | SR-27897 (CCK-A-Inhibitor, Sanofi-Synthelabo) | BCX-1777 (PNP-Inhibitor, BioCryst) |
| | Tocladesin (cyclisches-AMP Agonist, Ribapharm) | Ranpirnase (Ribonuclease-Stimulans, Alfacell) |
| | | |
| | Alvocidib (CDK-Inhibitor, Aventis) | Galarubicin (RNA-Synthese Inhibitor, Dong-A) |
| | CV-247 (COX-2-Inhibitor, Iv Medical) | Tirapazamin (Reduktionsmittel, SRI International) |
| | P54 (COX-2-Inhibitor, Phytopharm) | |
| | | N-Acetylcystein |
| | CapCell™ (CYP450- Stimulans, Bavarian Nordic) | (Reduktionsmittel Zambon) |
| | | R-Flurbiprofen (NF-kappaB-Inhibitor, Encore) |
| | GCS-IOO (gal3-Antagonist, GlycoGenesys) | |
| | | 3CPA (NF-kappaB-Inhibitor Active Biotech) |
| | G17DT-Immunogen (Gastrir Inhibitor, Aphton) | |
| | | Seocalcitol (Vitamin-D-Rezeptor-Agonist, Leo) |
| | Efaproxiral (Oxygenator, Alli Therapeutics) | |
| | | 131-I-TM-601 (DNA-Antagonist, TransMolecular |
| | PI-88 (Heparanase-Inhibitor Progen) | |
| | | Eflornithin (ODC-Inhibitor, ILEX Oncology) |
| | Tesmilifen (Histamin- | |
| | Antagonist, YM BioSciences | Minodronsäure (Osteoclaste Inhibitor, Yamanouchi) |
| | Histamin (Histamin-H2- | |
| | Rezeptor- Agonist, Maxim) | Indisulam (p53-Stimulans, Eisai) |
| | Tiazofurin (IMPDH-Inhibitor, Ribapharm) | |
| | | Aplidin (PPT-Inhibitor, PharmaMar) |
| | Cilengitid (Integrin-Antagoni Merck KGaA) | |
| | | Rituximab (CD20-Antikörpe Genentech) |
| | SR-31747 (IL-1-Antagonist, Sanofi-Synthelabo) | |
| | | Gemtuzumab (CD33-Antikörper, Wyeth Ayerst) |
| | CCI-779 (mTOR-Kinase-Inhibitor, Wyeth) | |
| | | PG2 (Hämatopoese-Verstärker, Pharmagenesis) |
| | Exisulind (PDE-V-Inhibitor, Cell Pathways) | |
| | | Immunol™ (Triclosan-Oralspülung, Endo) |
| | CP-461 (PDE-V-Inhibitor, C Pathways) | |
| | | Triacetyluridin (Uridin-Prodr Wellstat) |
| | AG-2037 (GART-Inhibitor, Pfizer) | |
| | | SN-4071 (Sarkom-Mittel, Signature BioScience) |
| | WX-UK1 (Plasminogenaktivator-Inhibitor, Wilex) | |
| | | TransMID-107™ (Immunotoxin, KS Biomedix |
| | | |
| | PBI-1402 (PMN-Stimulans, ProMetic LifeSciences) | PCK-3145 (Apoptose-Förderer, Procyon) |
| | | |
| | Bortezomib (Proteasom-Inhibitor, Millennium) | Doranidazol (Apoptose-Förderer, Pola) |
| | SRL-172 (T-Zell-Stimulans, SR Pharma) | CHS-828 (cytotoxisches Mittel, Leo) |
| | TLK-286 (Glutathion-S-Transferase-Inhibitor, Telik) | trans-Retinsäure (Differentiator, NIH) |
| | PT-100 (Wachstumsfaktor-Agonist, Point Therapeutics | MX6 (Apoptose-Förderer, MAXIA) |
| | Midostaurin (PKC-Inhibitor, Novartis) | Apomin (Apoptose-Förderer ILEX Oncology) |
| | Bryostatin-1 (PKC-Stimulan: GPC Biotech) | Urocidin (Apoptose-Fördere Bioniche) |
| | CDA-II (Apoptose-Förderer, Everlife) | Ro-31-7453 (Apoptose-Förderer, La Roche) |
| | SDX-101 (Apoptose-Fördere Salmedix) | Brostallicin (Apoptose-Förderer, Pharmacia) |
| | Ceflatonin (Apoptose-Förderer, ChemGenex) | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

### Messung der GSK3-beta Aktivität

Die Messung der GSK3-beta Aktivität kann analog WO 2008/078196 durchgeführt werden.
GSK3b (5-20 mU verdünnt in 20 mM MOPS ph 7.5, 1 mM EDTA, 0.01 % EDTA, 0.01 % Brj35, 5 % Glycerol, 0.1 % beta-Mercaptoethanol, 1 mg/ml BSA) wird gemessen gegen Phospho-GS2 Peptid
(YRRAAVPPSPSLSRHSSPHQS(PO4)EDEEE) in einem finalen Volumen von 25.5 µl enthaltend 8 mM MOPS ph 7.0, 0.2 mM EDTA, 20 µM Phospho GS2
Peptid, 10 mM Magnesiumacetat und 0.005 mM [33P-g-ATP] (50-1000 cpm/pmol) und wird 30 Minuten bei Raumtemperatur inkubiert. Der Assay wird durch Zusatz von 5 µl 0.5 M (3 %) Orthophosphorsäure gestoppt und dann wird auf P81 Unifilterplatten mit einem Waschpuffer (50 mM Orthophosphorsäure) geerntet.

### Messung der Met Kinase Aktivität

Die Met Kinase wird laut Herstellerangaben (Met, active, Upstate, Katalog-Nr. 14-526) zum Zweck der Proteinproduktion in Insektenzellen (Sf21; S. frugiperda) und der anschließenden affinitätschromatographischen Aufreinigung als "N-terminal 6His-tagged" rekombinantes humanes Protein in einem Baculovirus-Expressionsvektor exprimiert.

Zur Messung der Kinase-Aktivität kann auf verschiedene zur Verfügung stehender Meßsysteme zurückgegriffen werden. Beim Scintillation-Proximity-(Sorg et al., J. of. Biomolecular Screening, 2002, 7, 11-19), dem FlashPlate-Verfahren oder dem Filterbindungstest wird die radioaktive Phosphorylierung eines Proteins oder Peptids als Substrat mit radioaktiv markiertem ATP (³²P-ATP, ³³P-ATP) gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-Antikörper bindet nur das phosphorylierte Substrat. Diese Bindung ist mit einem zweiten Peroxidasekonjugierten Antikörper durch Chemilumineszenz nachweisbar (Ross et al., 2002, Biochem. J.).

### Flashplate-Verfahren (Met Kinase):

Als Testplatten dienen 96-well Flashplate^{R} Mikrotiterplatten der Firma Perkin Elmer (Kat.-Nr. SMP200). In die Assay Platte werden die Komponenten der unten beschriebenen Kinasereaktion pipettiert.
Die Met Kinase und das Substrat poly Ala-Glu-Lys-Tyr, (pAGLT, 6:2:5:1). werden mit radioaktiv markiertem ³³P-ATP in An- und Abwesenheit von Testsubstanzen in einem Gesamtvolumen von 100 µl bei Raumtemperatur 3 Std. inkubiert. Die Reaktion wird mit 150 µl einer 60mM EDTA-Lösung abgestoppt. Nach Inkubation für weitere 30 min bei Raumtemperatur werden die Überstände abgesaugt und die Wells dreimal mit je 200 µl 0,9% NaCl-Lösung gewaschen. Die Messung der gebundenen Radioaktivität erfolgt mittels eines Szintillationsmessgerätes (Topcount NXT, Fa. Perkin-Elmer). Als Vollwert wird die Inhibitor-freie Kinasereaktion verwendet. Dieser sollte ca. im Bereich von 6000-9000 cpm liegen. Als pharmakologischer Nullwert wird Staurosporin in einer Endkonzentration von 0,1 mM verwendet. Eine Bestimmung der Hemmwerte (IC50) erfolgt unter Verwendung des Programms RS1_MTS ().

Kinase-Reaktionsbedingungen pro well:
30 µl Assaypuffer
10 µl zu testende Substanz in Assaypuffer mit 10 % DMSO
10 µl ATP (Endkonzentration 1 µM kalt, 0,35 µCi ³³P-ATP)
50 µl Gemisch Met Kinase/Substrat in Assaypuffer;
   (10 ng Enzym/well, 50 ng pAGLT/well)

Verwendete Lösungen:
- Assay-Puffer:
   50 mM HEPES
   3 mM Magnesiumchlorid
   3 µM Natrium orthovanadat
   3 mM Mangan (II) chlorid
   1 mM Dithiothreitol (DTT)
   pH= 7,5 (einzustellen mit Natriumhydroxid)
- Stopp-Lösung:
   60 mM Titriplex III (EDTA)
- ³³P-ATP: Perkin-Elmer;
- Met Kinase: Upstate, Kat.-Nr. 14-526, Stock 1 µg/10 µl; spez. Aktivität 954 U/mg;
- Poly-Ala-Glu-Lys-Tyr, 6:2:5:1: Sigma Kat.-Nr. P1152

### In vivo-Tests

Experimenteller Ablauf: Weibliche Balb/C Mäuse (Züchter: Charles River Wiga) waren bei der Ankunft im Alter von 5 Wochen. Sie wurden 7 Tage lang an unsere Haltungsbedingungen akklimatisiert. Anschließend wurden jeder Maus 4 Millionen TPR-Met / NIH3T3 - Zellen in 100 µl PBS (ohne Ca++ und Mg++) subkutan im Beckenbereich injiziert. Nach 5 Tagen wurden die Tiere in 3 Gruppen randomisiert, so dass jede Gruppe von 9 Mäusen ein mittleres Tumorvolumen von 110 µl (Spanne: 55 - 165) hatte. Der Kontrollgruppe wurden 100 µl Vehikel (0,25 % Methylzellulose / 100 mM Acetatpuffer, pH 5.5), den Behandlungsgruppen wurden 200 mg/kg "A56" bzw. "A91" gelöst im Vehikel (Volumen ebenfalls 100 µl/ / Tier) per Schlundsonde täglich verabreicht. Nach 9 Tagen hatten die Kontrollen ein mittleres Volumen von 1530 µl und der Versuch wurde beendet.

Messung des Tumorvolumens: Die Länge (L) und Breite (B) wurde mit einer Schubleere gemessen und das Tumorvolumen nach der Formel LxBxB/2 berechnet.

Haltungsbedingungen: je 4 bzw. 5 Tiere pro Käfig, Fütterung mit kommerziellem Mäusefutter (Fa. Sniff).

Die Hemmung der SGK1 Proteinkinase kann im Filterbindungsverfahren bestimmt werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-lonisation) M⁺
FAB (Fast Atom Bombardment) (M+H)⁺
ESI (Electrospray lonization) (M+H)⁺ (wenn nichts anderes angegeben)

### HPLC-Methode

A (polar): Hewlett Pachard System der HP 1100 Serie mit den folgenden Merkmalen: lonenquelle: ES (positive mode), Scan 100-1000 m/z, Fragmentier-Spannung: 60 V, Gas-Temperatur: 300°C, DAD 220 nm
Säule: Chromolith SpeedROD RP-18e, 50-4.6
Flußrate 2.4 ml/min
Der verwendete Splitter reduzierte nach dem DAD die Flußrate für das MS auf 0,75 ml/min
Solvent A: Wasser +0.1% TFA
Solvent B: Acetonitiril + 0.08% TFA
Gradient:
0.0 min 5% B
2.8 min 100% B
3.3 min 100% B

B:
Säule: Chromolith SpeedROD RP-18e, 50-4.6
Flußrate 2.4 ml/min
Solvent A: Wasser +0.1 % TFA
Solvent B: Acetonitril + 0.1 % TFA
Gradient:
0.0 min 4% B
2.6 min 100% B
3.3 min 100% B

| | |
|---|---|
| **HPLC-MS-Methode** | : **Esi1.Rod.m / Polar.m / Unpolar.m** |
| **Säule** | : Chromolith Speed Rod RP 18e 50-4,6 mm |
| **Fluß** | : 2,4 ml/min |
| **Puffer A** | : 0,01 % TFA / Wasser |
| **Puffer B** | : 0,008% TFA / Acetonitril |
| **Wellenlänge** | : 220 nm |
| **Gradient Esi1.rod.m** | : 0,0-2,8min 20%-100% Puffer B; 2,8-3,3min 100% Puffer B; 3,3-3,4 min 100%-20% Puffer B; 3,4-3,8min 20 % Puffer B |
| **Gradient Polar.m** | : 0,0-3,0min 5%-100% Puffer B; 3,0-3,5min 100% Puffer B; 3,5-3,6 min 100%-5% Puffer B; 3,6-3,8min 20 % Puffer B |
| **Gradient Unpolar.m** | : 0,0-2,5min 40%-90% Puffer B; 2,5-3,8min 90% Puffer B; 3,8-3,9 min 90% Puffer B : 3,9-4,1min 90%-40 % Puffer B |

Abkürzungen:
DCM = Dichlormethan
EE = Essigsäureethylester
PE = Petrolether
RT = Raumtemperatur
DAPECI = N-(3-Dimethylaminopropyl)-N'-ethylcarbodiimidhydrochlorid
DMF = Dimethylformamid
HOBT = 1-Hydroxybenzotriazol
NCS = N-Chlorsuccinimid
TFA = Trifluoressigsäure

### Synthesebeispiele

Im Folgenden ist die Synthese für die Verbindungen "A1" bis "A10" aufgeführt.

### Methode 1: (Verbindungen "A1", "A2", "A4", "A5", "A6", "A7", "A8")

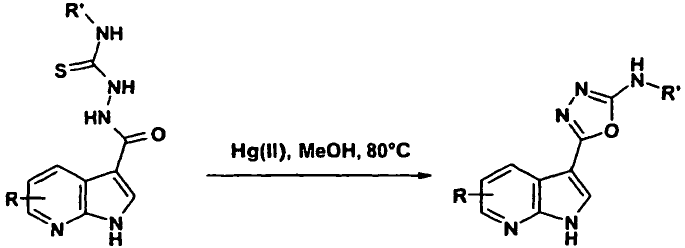

Die Oxadiazole werden durch Cyclisierung aus 4-substituierten 1-(1 H-Pyrrolo[2,3-b]pyridin-3-carbonyl)-4-phenylthiosemicarbaziden hergestellt, wobei das 1-(1H-Pyrrolo[2,3-b]pyridin noch weitere Substituenten an verschiedenen Positionen tragen kann.

### Herstellung von Phenyl-[5-(1H-pyrrolo[2,3-b]pyridin-3-yl)-[1,3,4]oxadiazol-2-yl]-amin ("A2")

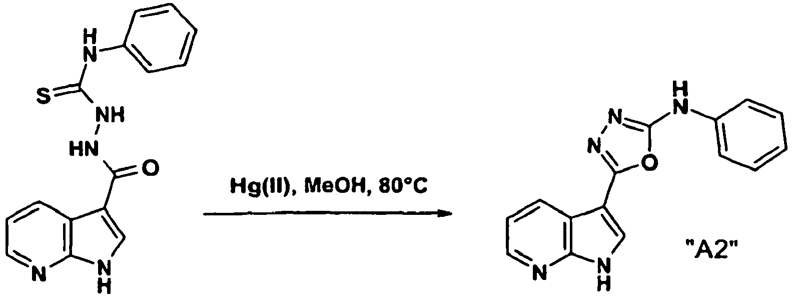

In einem verschlossenen Schraubdeckelgläschen werden 610 mg 1-(1 H-Pyrrolo[2,3-b]pyridin-3-carbonyl)-4-phenylthiosemicarbazid und 686 mg Quecksilber(II)acetat in 6 ml MeOH 2 Stunden bei 80°C gerührt. Zu der abgekühlten Reaktionslösung wird 1 Tropfen Na₂S-Lösung zugesetzt, alles über Kieselgur abgesaugt und mit warmem MeOH nachgewaschen.
Das Filtrat wird eingeengt und durch Säulenchromatographie mit (EE/Methanol, Gradient) an Kieselgel gereinigt. Man erhält 95 mg "A2" als farblosen Feststoff (Ausbeute 17,5 %); MS-FAB (M+H⁺) = 278,3; R_{f} (Methode polar): 1,72 Min.

Durch analoge Reaktionen werden die Verbindungen "A1", "A4", "A5", "A6", "A7", "A8" hergestellt:

| Verbindung Nr. | Name und/oder Struktur | MS-FAB (M+H⁺) / R_{f}-Wert |
|---|---|---|
| "A1" | 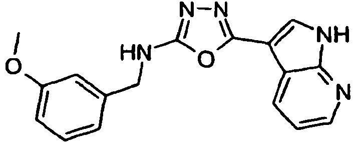 | 322, 34 / 1,61 min. (polar) |
| ¹H-NMR (400 MHz, DMSO-d₆, TFA-d₁) δ [ppm] 8,49-8,44 (2H, m), 8,31 (1H, s), 7,40 (1H, dd, J = 8,1 Hz, J = 5,1 Hz), 7,30 (1H, t, J = 8,0 Hz), 6,99-7,05 (2H, m), 6,88 (2H, dd, J = 8,0 Hz, J = 2,9 Hz ), 4,57 (2H, s), 3,76 (3H, s) | | |
| "A4" | 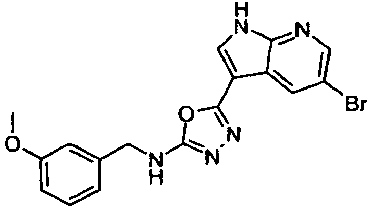 | 400,02; 402,02 / 1,96 min. (polar) |
| "A5" | 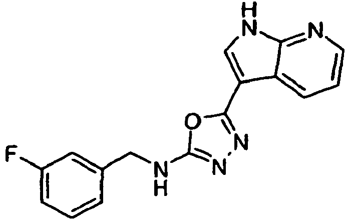 | 310,30/ 1,67 min. (polar) |
| "A6" | 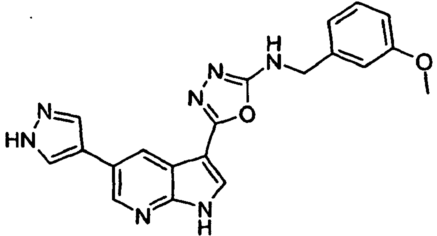 | 388,40 / 2,13 min. (polar) |
| "A7" | 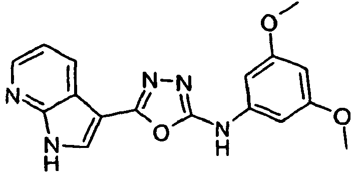 | 338,34 / 1,77 min. (polar) |
| "A8" | 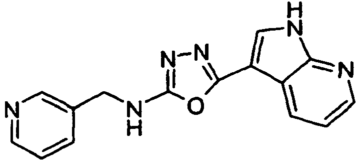 | 293,30 / 0,96 min. (polar) |

Die für die Cyclisierung benötigten substituierten Thiosemicarbazide sind durch dem Fachmann bekannte Methoden zugänglich, z.B. durch die beiden folgenden Synthesesequenzen B1 und B2:

### Methode B1 (für "A2", "A4", "A6", "A7")

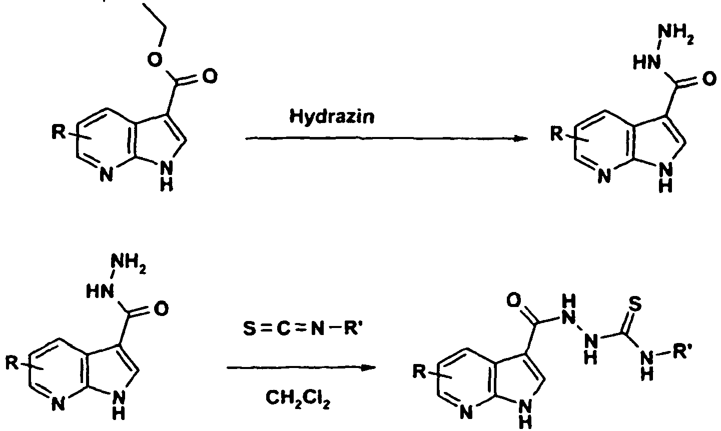

Beispiel für Schritt 1: Darstelllung der Hydrazide (z.B. Vorstufe 1 für "A2")

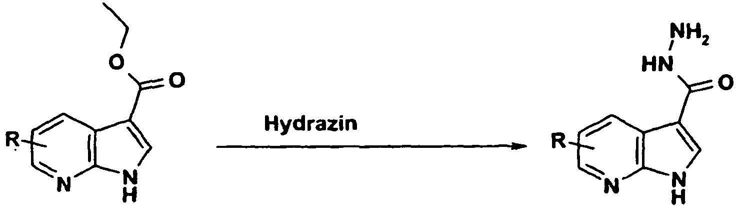

In einem Reaktionsgefäß werden 1,40 g 1-(1H-Pyrrolo[2,3-b]pyridin-3-carbonsäure. 3,8 ml Hydrazinhydrat in 1 ml Dioxan 12 Stunden bei 100°C gerührt. Beim Abkühlen fällt ein farbloser Feststoff an, der abgesaugt, mit Wasser gewaschen und getrocknet wird. Man erhält 1,05 g 1-(1 H-Pyrrolo[2,3-b]pyridin-3-carbonhydrazid (71% Ausbeute); MS-FAB (M+H⁺) = 177,1; R_{f} (Methode polar): 0,70 Min.

Beispiele für eingesetzte 7-Azaindol-carbonsäureester.

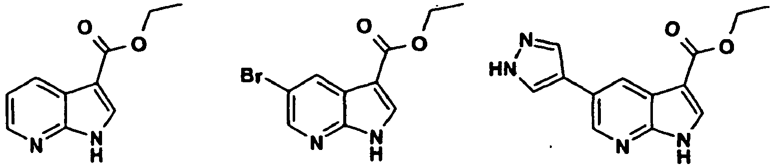

Analog können auch andere substituierte 7-Azaindol-3-carbonsäureester eingesetzt werden (vorzugweise Methyl- oder Ethylester).

### Beispiel für Schritt 2: Darstellung der Thiosemicarbazide (z.B. Vorstufe 2 für "A2"

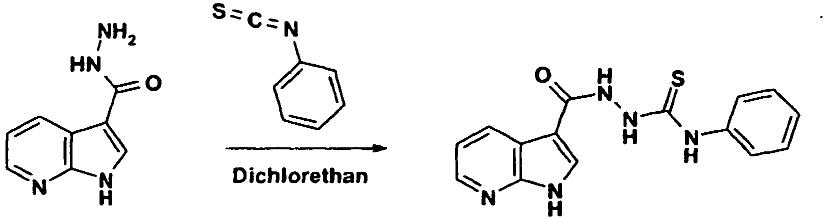

In einem Schraubdeckelgläschen werden 350 mg (1H-Pyrrolo[2,3-b]pyridin-3-carbosäurehydrazid und 261 µl Phenylisothiocyanat in 10 ml Dichlorethan bei 60°C 15 Stunden gerührt. Es werden weitere 78 µl Phenylisothiocyanat zugegeben und bei derselben Temperatur 20 Stunden gerührt. Nach dem Abkühlen im Kühlschrank saugt man den Niederschlag ab, trocknet und erhält 610 mg 1-(1H-Pyrrolo[2,3-b]pyridine-3-carbonyl)-4-phenylthiosemicarbazid als farbloses Pulver; (Ausbeute 98,6 %); MS-FAB (M+H⁺) = 312,3; R_{f} (Methode polar): 1,43 Min.

Beispiele für eingesetzte Isothiocyanate:

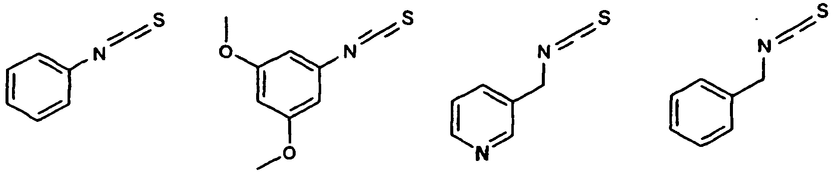

Analog können auch andere kommerziell erhältliche Verbindungen eingesetzt werden.

### Methode B2 (angewendet für "A1", "A4", A5" und "A8")

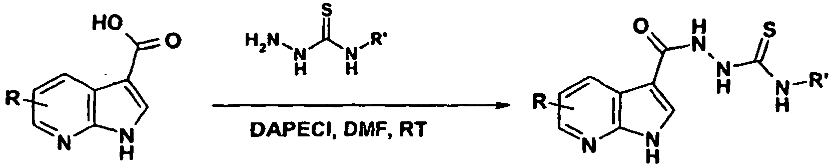

### Beispiel

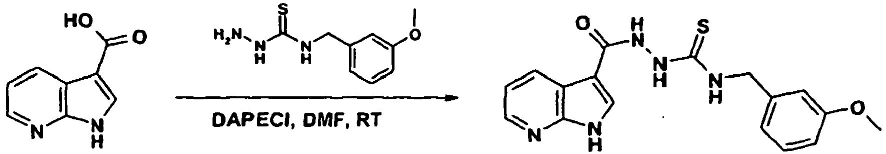

In einem Schraubdeckelgläßchen werden 200 mg 1-(1H-Pyrrolo[2,3-b]pyridin-3-carbonsäure, 260,5 mg 4-(3-Methoxybenzyl)thiosemicarbazid, 230 mg HOBT und 288 mg DAPECI in 3 ml DMF 3 Tage bei RT gerührt. Der Ansatz wird auf Wasser gegossen und der entstandene Niederschlag abgesaugt und getrocknet. Man erhält 250 mg Kupplungsprodukt [57%, MS-FAB (M+H⁺) = 356,4; R_{f} (Methode polar): 1,60 Min], das ohne weitere Reinigung für die Cyclisierung eingesetzt werden kann.

Weitere Beispiele für eingesetzte Bausteine:

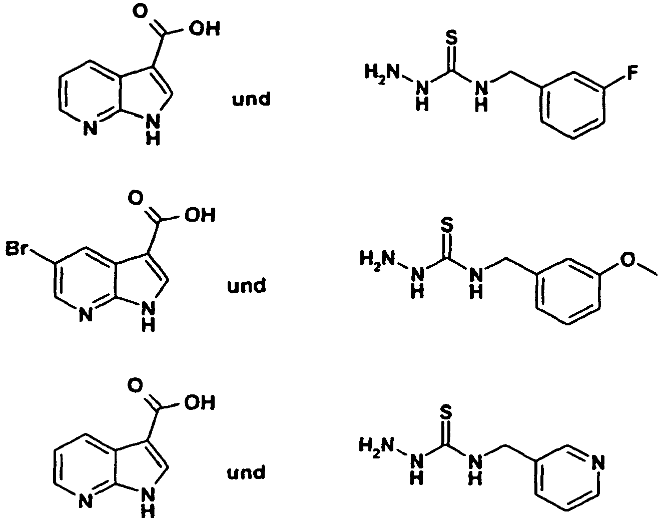

Analog können auch andere substituierte 7-Azaindol-3-carbonsäuren und substituierte Thiosemicarbazide eingesetzt werden.

### Methode 2: (Herstellung von 5-(1H-Pyrrolo[2,3-b]pyridin-3-yl)-[1,3,4]oxadiazol-2-ylamin "A3")

### Beispiel:

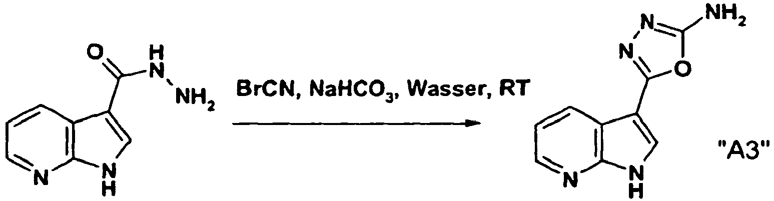

Zu einer Mischung von 500 mg 1-(1H-Pyrrolo[2,3-b]pyridin-3-carbonsäurehydrazid, 1,192 g Natriumhydrogencarbonat in 50 ml Wasser und 5 ml DMF wird unter Rühren 360,8 mg Bromcyan zugetropft. Die Reaktionsmischung fängt an zu schäumen und ein feiner Niederschlag bildet sich. Diese wird nach 2 Stunden abgesaugt und getrocknet. Man erhält 450 mg 5-(1H-Pyrrolo[2,3-b]pyridin-3-yl)-[1,3,4]oxadiazol-2-ylamin (Ausbeute: 78,8 %); MS-FAB (M+H⁺) = 202,2; R_{f} (Methode polar): 1,00 Min;
¹H-NMR (250 MHz, DMSO-d, TFA-d₁) δ [ppm] 8,56-8,61 (2H, m), 8,38 (1H, s), 7,52 (1H, dd, J = 8,1 Hz, J = 5,1 Hz).

### Methode 3: (Verbindungen "A9", "A10")

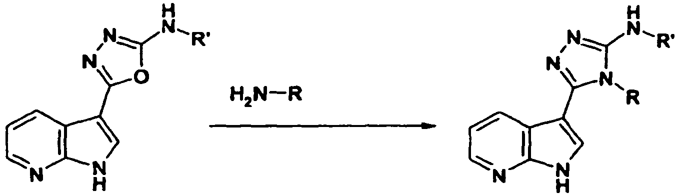

### Herstellung von N'3'-(3-Methoxy-benzyl)-5-(1H-pyrrolo[2,3-b]pyridin-3-yl)-[1,2,4]triazole-3,4-diamin ("A10")

In einem geschlossenen Schraubdeckgläschen wird eine Mischung aus 50 mg (3-Methoxy-benzyl)-[5-(1H-pyrrolo[2,3-b]pyridin-3-yl)-[1,3,4]oxadiazol-2-yl]-amin (hergestellt nach Methode 1) und 75 µl Hydrazinhydrat in 1 ml 2-Propanol bei 100°C-120°C Badtemperatur 5 Tage gerührt. Die Reaktionsmischung wird eingeengt und durch präp. HPLC gereinigt (RP-18, Wasser/Acetonitril).
Man erhält 16,6 mg N*3*-(3-Methoxy-benzyl)-5-(1H-pyrrolo[2,3-b]pyridin-3-yl)-[1,2,4]triazole-3,4-diamin (31,8%) MS-FAB (M+H⁺) = 336,4 R_{f} (Methode polar): 1,34 Min;
¹H-NMR (400 MHz, DMSO-d₆, TFA-d₁) δ [ppm] 8,69 (1H, s), 8,64 (1H, d, J = 8,1 Hz), 8,53 (1H, dd, J = 5,1 Hz, J = 1,7 Hz), 7,45 (1H, dd, J = 8,1 Hz, J = 5,1 Hz), 7,33 (1H, t, J = 8,0 Hz), 6,02-7,09 (2H, m), 6,91 (1H, dd, J = 8,0 Hz, J = 2,9 Hz ), 4,59 (2H, s), 3,79 (3H, s);

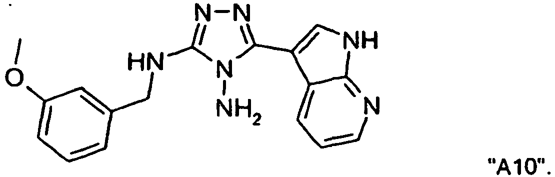

### Beispiel: Herstellung von [4-Benzyl-5-(1H-pyrrolo[2,3-b]pyridin-3-yl-4H-[1,2,4]triazol-3-yl]-(3-methoxy-benzyl)-amin ("A9"):

Analog kann die Verbindung "A9" durch Verwendung von Benzylamin statt Hydrazinhydrat hergestellt werden, wobei als Lösungsmittel 1-Butanol verwendet wird und die Reaktionszeit 6 Tage beträgt; 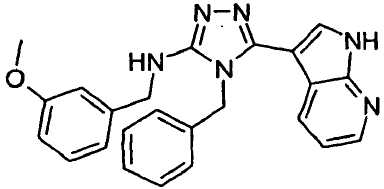 "A9", R_{f} 1,60 min. (polar);
MS-FAB (M+H⁺) = 411,48.

Pharmakologische Daten

**Tabelle 1**

| Verbindung Nr. | Target | Inhibierung IC₅₀ (Enzym) | |
|---|---|---|---|
| "A1" | SGK1 | A | |
| | TGF-beta | B | |
| | MKK1 | A | |
| | SAPK3 | B | |
| | AMPK | A | |
| | CHK2 | A | |
| | GSK3-beta | A | |
| "A2" | SGK1 | B | |
| "A3" | SGK1 | B | |
| "A4" | SGK1 | A | |
| | Met-Kinase | B | |
| "A5" | SGK1 | A | |
| | TGF-beta | B | |
| | Met-Kinase | B | |
| "A6" | SGK1 | B | |
| "A7" | | | |
| "A8" | TGF-beta | B | |
| "A9" | SGK1 | B | |
| "A10" | SGK1 | B | |

| | | | |
|---|---|---|---|
| IC₅₀: 1 nM - 0,1 µM = A 0,1 µM-10µM=B > 10 µM = C | | | |

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 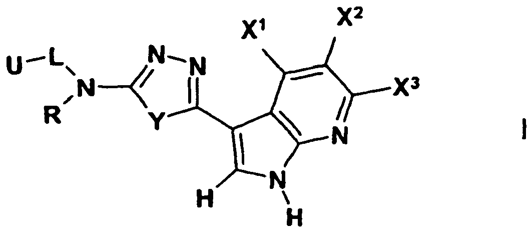 worin
L fehlt, CR⁷R⁸, CR⁷R⁸CR⁹R¹⁰, CR⁷R⁸C(OR⁹)R¹⁰, NR⁷, O, NR⁶CR⁷R⁸, CR⁷R⁸NR⁹, OCR⁷R⁸, OCR⁷R⁸CR⁹R¹⁰, CR⁷R⁸O, CR⁷R⁸CR⁹R¹⁰O, NR⁶CR⁷R⁸CR⁹R¹⁰, CR⁷R⁸SO₂, NR⁷CONR⁸, NR⁷CONR⁸CR⁹R¹⁰, COCR⁷R⁸, CONR⁷, CONR⁷CR⁸R⁹, CONHNH, NR⁷CR⁸R⁹CONR¹⁰, NR⁷CO oder NR⁷COCR⁸R⁹,
U H, A, Ar oder Het,
Y O, NH, NNH₂ oder N-[C(R⁷)₂]ₙAr,
R H oder R¹¹,
X¹, X², X³ jeweils unabhängig voneinander H, A, Hal, OH, OA, -[C(R⁷)₂]ₙAr, -[C(R⁷)₂]ₙHet, OAr, OHet, SH, SA, SAr, SHet, NH₂, NHA, NAA', NHAr, N(Ar)₂, NHHet, N(Het)₂, NAAr, NAHet, SOA, SOAr, SOHet, SO₂A, SO₂Ar, SO₂Het, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr, COHet, SO₃H, SO₂NH₂, SO₂NHAr, SO₂N(Ar)₂, SO₂NHHet oder SO₂N(Het)₂,
R⁶, R⁷, R⁸, R⁹, R¹⁰ R¹¹ jeweils unabhängig voneinander H oder A, Alkyl mit 1-6 C-Atomen, worin 1-5 H-Atome durch F ersetzt sein können,
A, A' jeweils unabhängig voneinander unsubstituiertes oder ein-, zwei- oder dreifach durch =S, =NR⁷ und/oder =O (Carbonylsauerstoff) substituiertes Alkyl mit 1-10 C-Atomen, worin eine, zwei oder drei CH₂-Gruppen durch O, S, SO, SO₂, NH, NR¹¹ und/oder durch -CH=CH-Gruppen und/oder auch 1-7 H-Atome durch F und/oder Cl ersetzt sein können, oder cyclisches Alkyl mit 3-7 C-Atomen,
Ar unsubstituiertes oder ein-, zwei-, drei-oder vierfach durch A, Hal, OH, OA, Ar', OAr', Het, OHet, SH, SA, SAr', SHet, NH₂, NHA, NAA', NHAr', N(Ar')₂, NHHet, N(Het)₂, NAAr', NAHet, SOA, SOAr', SOHet, SO₂A, SO₂Ar', SO₂Het, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', COHet, SO₃H, SO₂NH₂, SO₂NHAr', SO₂N(Ar')₂. SO₂NHHet und/oder SO₂N(Het)₂ substituiertes Phenyl, Naphthyl oder Biphenyl,
Het einen ein- oder zweikernigen gesättigten, ungesättigten oder aromatischen Heterocyclus mit 1 bis 4 N-, O- und/oder S-Atomen, der ein-, zwei- oder dreifach durch A, Hal, OH, OA, Ar, OAr, Het', OHet', SH, SA, SAr', SHet', NH₂, NHA, NAA', NHAr', N(Ar')₂, NHHet', N(Het')₂, NAAr', NAHet', SOA, SOAr', SOHet', SO₂A, SO₂Ar', SO₂Het', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', COHet', SO₃H, SO₂NH₂, SO₂NHAr, SO₂N(Ar')₂, SO₂NHHet' oder SO₂N(Het')₂. =S, =NR⁷ und/oder =O (Carbonylsauerstoff) substituiert sein kann,
Ar' unsubstituiertes oder ein-, zwei-, drei-oder vierfach durch A, Hal, OH, OA, OPhenyl, SH, SA, NH₂, NHA, NAA', NHPhenyl, SOA, SOPhenyl, SO₂A, SO₂Phenyl, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COPhenyl, SO₃H, SO₂NH₂, SO₂NHPhenyl und/oder SO₂N(Phenyl)₂ substituiertes Phenyl,
Het' einen ein- oder zweikernigen gesättigten, ungesättigten oder aromatischen Heterocyclus mit 1 bis 4 N-, O- und/oder S-Atomen, der ein-, zwei- oder dreifach durch A, Hal, OH, OA, NH₂, NHA, NAA', SOA, SOAr', SO₂A, SO₂Ar', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', SO₃H, SO₂NH₂, SO₂NHAr', SO₂N(Ar')₂, =S, =NR⁷ und/oder =O (Carbonylsauerstoff) substituiert sein kann,
Hal F, Cl, Br oder I,
*n* 0, 1 oder 2,
bedeuten,
sowie ihre pharmazeutisch verwendbaren Salze und Stereoisomere,
einschließlich deren Mischungen in allen Verhältnissen.

2. Verbindungen nach Anspruch 1, worin
L fehlt oder CR⁷R⁸ bedeutet,
sowie ihre pharmazeutisch verwendbaren Salze und Stereoisomere,
einschließlich deren Mischungen in allen Verhältnissen.

3. Verbindungen nach Anspruch 1 oder 2, worin
R H bedeutet,
sowie ihre pharmazeutisch verwendbaren Salze und Stereoisomere,
einschließlich deren Mischungen in allen Verhältnissen.

4. Verbindungen nach einem oder mehreren der Ansprüche 1-3, worin
A Alkyl mit 1-10 C-Atomen, worin 1-7 H-Atome durch F und/oder Cl ersetzt sein können,
bedeutet,
sowie ihre pharmazeutisch verwendbaren Salze und Stereoisomere,
einschließlich deren Mischungen in allen Verhältnissen.

5. Verbindungen nach einem oder mehreren der Ansprüche 1-4, worin
Ar unsubstituiertes oder ein-, zwei-, drei- oder vierfach durch A, Hal, OH und/oder OA substituiertes Phenyl,
bedeutet,
sowie ihre pharmazeutisch verwendbaren Salze und Stereoisomere,
einschließlich deren Mischungen in allen Verhältnissen.

6. Verbindungen nach einem oder mehreren der Ansprüche 1-5, worin
Het einen einkernigen aromatischen Heterocyclus mit 1 bis 4 N-, O- und/oder S-Atomen,
bedeutet,
sowie ihre pharmazeutisch verwendbaren Salze und Stereoisomere,
einschließlich deren Mischungen in allen Verhältnissen.

7. Verbindungen nach einem oder mehreren der Ansprüche 1-6, worin
Het 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4-oder 5-Isothiazolyl, 2-, 3- oder 4-Pyridyl, 2-, 4-, 5- oder 6-Pyrimidinyl, 1,2,3-Triazol-1-, -4- oder-5-yl, 1,2,4-Triazol-1-, -3-oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3-oder 4-Pyridazinyl oder Pyrazinyl,
bedeutet,
sowie ihre pharmazeutisch verwendbaren Salze und Stereoisomere,
einschließlich deren Mischungen in allen Verhältnissen.

8. Verbindungen nach einem oder mehreren der Ansprüche 1-7, worin
R⁷, R⁸ jeweils unabhängig voneinander H oder R¹¹,
bedeuten,
sowie ihre pharmazeutisch verwendbaren Salze und Stereoisomere,
einschließlich deren Mischungen in allen Verhältnissen.

9. Verbindungen nach einem oder mehreren der Ansprüche 1-8, worin
R⁷, R⁸ jeweils unabhängig voneinander H oder CH₃,
bedeuten,
sowie ihre pharmazeutisch verwendbaren Salze und Stereoisomere,
einschließlich deren Mischungen in allen Verhältnissen.

10. Verbindungen nach einem oder mehreren der Ansprüche 1-9, worin
Het Pyrrolyl, 2-, 3- oder 4-Pyridyl, 2- oder 3-Furyl oder 2- oder 3-Thienyl
bedeutet,
sowie ihre pharmazeutisch verwendbaren Salze und Stereoisomere,
einschließlich deren Mischungen in allen Verhältnissen.

11. Verbindungen nach einem oder mehreren der Ansprüche 1-10, worin
X¹, X², X³ jeweils unabhängig voneinander H, Hal oder -[C(R⁷)₂]ₙHet,
bedeuten,
sowie ihre pharmazeutisch verwendbaren Salze und Stereoisomere,
einschließlich deren Mischungen in allen Verhältnissen.

12. Verbindungen nach einem oder mehreren der Ansprüche 1-11, worin
L fehlt oder CR⁷R⁸,
U H, A, Ar oder Het,
Y O, NH, NNH₂ oder N-[C(R7)₂]ₙAr,
R H oder R¹¹,
X¹, X², X³ jeweils unabhängig voneinander H, Hal oder -[C(R7)₂]ₙHet,
R⁷, R⁸ jeweils unabhängig voneinander H oder R¹¹,
R¹¹ Alkyl mit 1-6 C-Atomen, worin 1-5 H-Atome durch F ersetzt sein können,
A Alkyl mit 1-10 C-Atomen, worin 1-7 H-Atome durch F und/oder Cl ersetzt sein können,
Ar unsubstituiertes oder ein-, zwei-, drei- oder vierfach durch A, Hal, OH und/oder OA substituiertes Phenyl,
Het einen einkernigen aromatischen Heterocyclus mit 1 bis 4 N-, O- und/oder S-Atomen,
Hal F, Cl, Br oder I,
n 0, 1 oder 2,
bedeuten,
sowie ihre pharmazeutisch verwendbaren Salze und Stereoisomere,
einschließlich deren Mischungen in allen Verhältnissen.

13. Verbindungen nach Anspruch 1 ausgewählt aus der Gruppe
| Nr. | Strukturformel und/oder Name |
|---|---|
| "A1" | 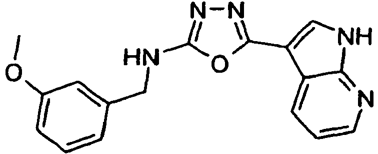 |
| "A2" | Phenyl-[5-(1H-pyrrolo[2,3-b]pyridin-3-yl)-[1,3,4]oxadiazo 2-yl]-amin |
| "A3" | 5-(1H-Pyrrolo[2,3-b]pyridin-3-yl)-[1,3,4]oxadiazol-2-ylam |
| "A4" | 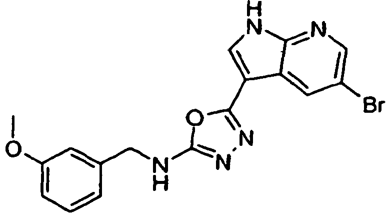 |
| "A5" | 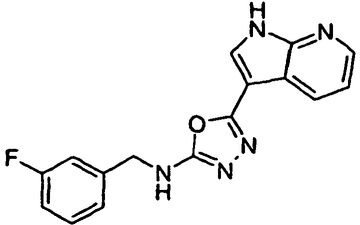 |
| "A6" | 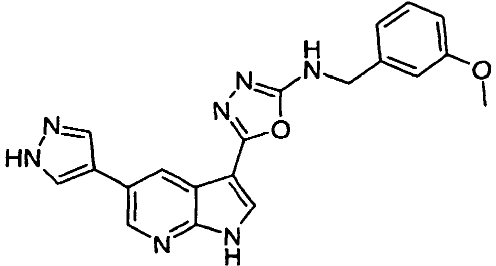 |
| "A7" | 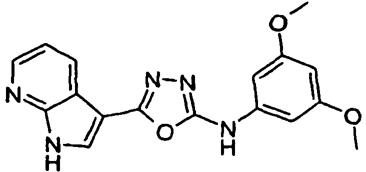 |
| "A8" | 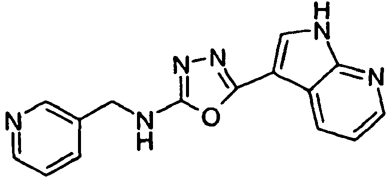 |
| "A9" | 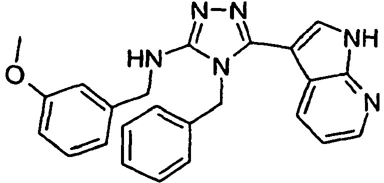 |
| "A10" | 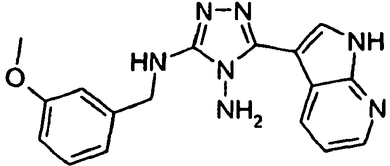 |
sowie ihre pharmazeutisch verwendbaren Salze und Stereoisomere, einschließlich deren Mischungen in allen Verhältnissen.

14. Verfahren zur Herstellung von Verbindungen der Formel I nach den Ansprüchen 1-13 sowie ihrer pharmazeutisch verwendbaren Salze und Stereoisomere, **dadurch gekennzeichnet, daß** man
a) eine Verbindung der Formel II 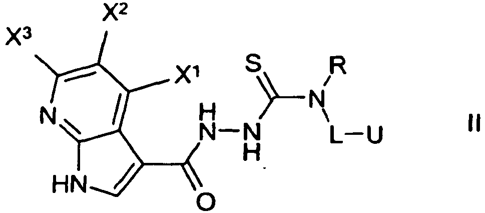 worin X¹, X², X³, R, L und U die in Anspruch 1 angegebenen Bedeutungen haben,
cyclisiert,
oder
b) eine Verbindung der Formel III 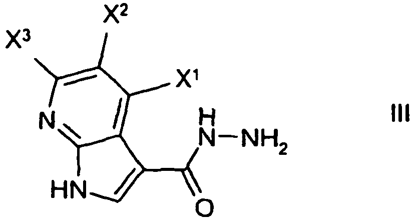 worin X¹, X², X³ die in Anspruch 1 angegebenen Bedeutungen haben,
mit einem Cyanhalogenid umsetzt,
oder
c) eine Verbindung der Formel I, worin Y Sauerstoff bedeutet, mit einem Hydrazinderivat oder mit H₂N-[C(R⁷)₂]ₙAr zu einer Verbindung der Formel I umwandelt, worin Y NNH₂ oder N-[C(R⁷)₂]ₙAr bedeutet,
und/oder eine Base oder Säure der Formel I in eines ihrer Salze umwandelt.

15. Arzneimittel, enthaltend mindestens eine Verbindung nach Anspruch 1-13 und/oder ihre pharmazeutisch verwendbaren Salze und Stereoisomere, einschließlich deren Mischungen in allen Verhältnissen, sowie gegebenenfalls Träger- und/oder Hilfsstoffe.

16. Verwendung von Verbindungen gemäß Anspruch 1-13, sowie ihrer pharmazeutisch verwendbaren Salze und Stereoisomere, einschließlich deren Mischungen in allen Verhältnissen, zur Herstellung eines Arzneimittels zur Behandlung von Krankheiten, die durch Inhibierung von Proteinkinasen durch die Verbindungen nach Anspruch 1-13 beeinflußt werden.

17. Verwendung nach Anspruch 16 von Verbindungen gemäß Anspruch 1 - 13, sowie ihrer pharmazeutisch verwendbaren Salze und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress und zur Behandlung von Tinitus.

18. Verwendung nach Anspruch 16 von Verbindungen gemäß Anspruch 1-13, sowie ihrer pharmazeutisch verwendbaren Salze und Stereoisomere, einschließlich deren Mischungen in allen Verhältnissen, zur Herstellung eines Arzneimittels zur Behandlung oder Vorbeugung von neurodegenerativen Erkrankungen, Parkinson, Tauopathien, Morbus Alzheimer, corticobasale Degeneration, Morbus Pick, Morbus Wilson, Morbus Huntington, vaskuläre Dementia, akuter Schlaganfall, periphere Neuropathien, Retinipathie oder Glaucom, manisch depressive Erkrankungen, Autoimmunerkrankungen, inflammatorischen und proliferativen Erkrankungen, AIDS, Asthma, Rhinitis, Morbus Crohn.

19. Verwendung nach Anspruch 16 von Verbindungen gemäß Anspruch 1-13, sowie ihrer pharmazeutisch verwendbaren Salze und Stereoisomere, einschließlich deren Mischungen in allen Verhältnissen, zur Herstellung eines Arzneimittels wobei die zu behandelnde Krankheit ein fester Tumor ist.

20. Verwendung nach Anspruch 19, wobei der feste Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge stammt.

21. Verwendung nach Anspruch 19, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom stammt.

22. Verwendung nach Anspruch 20, wobei der feste Tumor aus der Gruppe der Lungenadenokarzinom, kleinzellige Lungenkarzinome, Bauchspeicheldrüsenkrebs, Glioblastome, Kolonkarzinom und Brustkarzinom stammt.

23. Verwendung nach Anspruch 17, wobei die zu behandelnde Krankheit ein Tumor des Blut- und Immunsystems ist.

24. Verwendung nach Anspruch 23, wobei der Tumor aus der Gruppe der akuten myelotischen Leukämie, der chronischen myelotischen Leukämie, akuten lymphatischen Leukämie und/oder chronischen lymphatischen Leukämie stammt.

25. Arzneimittel enthaltend mindestens eine Verbindung gemäß Anspruch 1-13 und/oder ihre pharmazeutisch verwendbaren Salze und Stereoisomere, einschließlich deren Mischungen in allen Verhältnissen, und mindestens einen weiteren Arzneimittelwirkstoff.

26. Set (Kit), bestehend aus getrennten Packungen von
(a) einer wirksamen Menge an einer Verbindung gemäß Anspruch 1-13 und/oder ihrer pharmazeutisch verwendbaren Salze und Stereoisomere, einschließlich deren Mischungen in allen Verhältnissen, und
(b) einer wirksamen Menge eines weiteren Arzneimittelswirkstoffs.

## Claims

1. Compounds of the formula I 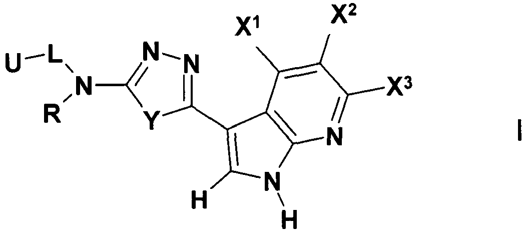 in which
L is absent or denotes CR⁷R⁸, CR⁷R⁸CR⁹R¹⁰, CR⁷R⁸C(OR⁹)R¹⁰, NR⁷, O, NR⁶CR⁷R⁸, CR⁷R⁸NR⁹, OCR⁷R⁸, OCR⁷R⁸CR⁹R¹⁰ , CR⁷R⁸O CR⁷R⁸CR⁹R¹⁰O, NR⁶CR⁷R⁸CR⁹R¹⁰ CR⁷R⁸SO₂, NR⁷CONR⁸, NR⁷CONR⁸CR⁹R¹⁰, COCR⁷R⁸, CONR⁷, CONR⁷CR⁸R⁹, CONHNH, NR⁷CR⁸R⁹CONR¹⁰, NR⁷CO or NR⁷COCR⁸R⁹,
U denotes H, A, Ar or Het,
Y denotes O, NH, NNH₂ or N-[C(R⁷)₂]ₙAr,
R denotes H or R¹¹,
X¹, X², X³ each, independently of one another, denote H, A, Hal, OH, OA, -[C(R⁷)₂]ₙAr, -[C(R⁷)₂]ₙHet, OAr, OHet, SH, SA, SAr, SHet, NH₂, NHA, NAA', NHAr, N(Ar)₂, NHHet, N(Het)₂, NAAr, NAHet, SOA, SOAr, SOHet, SO₂A, SO₂Ar, SO₂Het, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr, COHet, SO₃H, SO₂NH₂, SO₂NHAr, SO₂N(Ar)₂, SO₂NHHet or SO₂N(Het)₂,
R⁶ R⁷, R⁸, R⁹, R¹⁰ R¹¹ each, independently of one another, denote H or A, denotes alkyl having 1-6 C atoms, in which 1-5 H atoms may be replaced by F,
A, A' each, independently of one another, denote alkyl having 1-10 C atoms which is unsubstituted or mono-, di- or trisubstituted by =S, =NR⁷ and/or =O (carbonyl oxygen) and in which one, two or three CH₂ groups may be replaced by O, S, SO, SO₂, NH, NR¹¹ and/or by -CH=CH- groups and/or, in addition, 1-7 H atoms may be replaced by F and/or Cl, or cyclic alkyl having 3-7 C atoms,
Ar denotes phenyl, naphthyl or biphenyl, each of which is unsubstituted or mono-, di-, tri- or tetrasubstituted by A, Hal, OH, OA, Ar', OAr', Het, OHet, SH, SA, SAr', SHet, NH₂, NHA, NAA', NHAr', N(Ar')₂, NHHet, N(Het)₂, NAAr', NAHet, SOA, SOAr', SOHet, SO₂A, SO₂Ar', SO₂Het, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', COHet, SO₃H, SO₂NH₂, SO₂NHAr', SO₂N(Ar')₂, SO₂NHHet and/or SO₂N(Het)₂,
Het denotes a mono- or bicyclic saturated, unsaturated or aromatic heterocycle having 1 to 4 N, O and/or S atoms, which may be mono-, di- or trisubstituted by A, Hal, OH, OA, Ar, OAr, Het', OHet', SH, SA, SAr', SHet', NH₂, NHA, NAA', NHAr', N(Ar')₂, NHHet', N(Het')₂, NAAr', NAHet', SOA, SOAr', SOHet', SO₂A, SO₂Ar', SO₂Het', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', COHet', SO₃H, SO₂NH₂, SO₂NHAr', SO₂N(Ar')₂, SO₂NHHet' or SO₂N(Het')₂, =S, =NR⁷ and/or =O (carbonyl oxygen),
Ar' denotes phenyl which is unsubstituted or mono-, di-, tri- or tetrasubstituted by A, Hal, OH, OA, O-phenyl, SH, SA, NH₂, NHA, NAA', NH-phenyl, SOA, SO-phenyl, SO₂A, SO₂-phenyl, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, CO-phenyl, SO₃H, SO₂NH₂, SO₂NH-phenyl and/or SO₂N(phenyl)₂,
Het' denotes a mono- or bicyclic saturated, unsaturated or aromatic heterocycle having 1 to 4 N, O and/or S atoms, which may be mono-, di- or trisubstituted by A, Hal, OH, OA, NH₂, NHA, NAA', SOA, SOAr', SO₂A, SO₂Ar', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', SO₃H, SO₂NH₂, SO₂NHAr', SO₂N(Ar')₂, =S, =NR⁷ and/or =O (carbonyl oxygen),
Hal denotes F, Cl, Br or I,
n denotes 0, 1 or 2,
and pharmaceutically usable salts and stereoisomers thereof, including mixtures thereof in all ratios.

2. Compounds according to Claim 1 in which
L is absent or denotes CR⁷R⁸,
and pharmaceutically usable salts and stereoisomers thereof, including mixtures thereof in all ratios.

3. Compounds according to Claim 1 or 2 in which
R denotes H,
and pharmaceutically usable salts and stereoisomers thereof, including mixtures thereof in all ratios.

4. Compounds according to one or more of Claims 1-3 in which
A denotes alkyl having 1-10 C atoms, in which 1-7 H atoms may be replaced by F and/or Cl,
and pharmaceutically usable salts and stereoisomers thereof, including mixtures thereof in all ratios.

5. Compounds according to one or more of Claims 1-4 in which
Ar denotes phenyl which is unsubstituted or mono-, di-, tri- or tetrasubstituted by A, Hal, OH and/or OA,
and pharmaceutically usable salts and stereoisomers thereof, including mixtures thereof in all ratios.

6. Compounds according to one or more of Claims 1-5 in which
Het denotes a monocyclic aromatic heterocycle having 1 to 4 N, O and/or S atoms,
and pharmaceutically usable salts and stereoisomers thereof, including mixtures thereof in all ratios.

7. Compounds according to one or more of Claims 1-6 in which
Het denotes 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or 5-yl, 1- or -5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4-or -5-yl, 3- or 4-pyridazinyl or pyrazinyl,
and pharmaceutically usable salts and stereoisomers thereof, including mixtures thereof in all ratios.

8. Compounds according to one or more of Claims 1-7 in which R⁷, R⁸ each, independently of one another, denote H or R¹¹, and pharmaceutically usable salts and stereoisomers thereof, including mixtures thereof in all ratios.

9. Compounds according to one or more of Claims 1-8 in which R⁷, R⁸ each, independently of one another, denote H or CH₃, and pharmaceutically usable salts and stereoisomers thereof, including mixtures thereof in all ratios.

10. Compounds according to one or more of Claims 1-9 in which
Het denotes pyrrolyl, 2-, 3- or 4-pyridyl, 2- or 3-furyl or 2- or 3-thienyl,
and pharmaceutically usable salts and stereoisomers thereof, including mixtures thereof in all ratios.

11. Compounds according to one or more of Claims 1-10 in which X¹, X², X³ each, independently of one another, denote H, Hal or -[C(R⁷)₂]ₙHet,
and pharmaceutically usable salts and stereoisomers thereof, including mixtures thereof in all ratios.

12. Compounds according to one or more of Claims 1-11 in which
L is absent or denotes CR⁷R⁸,
U denotes H, A, Ar or Het,
Y denotes O, NH, NNH₂ or N-[C(R⁷)₂]ₙAr,
R denotes H or R¹¹,
X¹, X², X³ each, independently of one another, denote H, Hal or -[C(R⁷)₂]ₙHet,
R⁷, R⁸ each, independently of one another, denote H or R¹¹,
R¹¹ denotes alkyl having 1-6 C atoms, in which 1-5 H atoms may be replaced by F,
A denotes alkyl having 1-10 C atoms, in which 1-7 H atoms may be replaced by F and/or Cl,
Ar denotes phenyl which is unsubstituted or mono-, di-, tri- or tetrasubstituted by A, Hal, OH and/or OA,
Het denotes a monocyclic aromatic heterocycle having 1 to 4 N, O and/or S atoms,
Hal denotes F, Cl, Br or I,
n denotes 0, 1 or 2,
and pharmaceutically usable salts and stereoisomers thereof, including mixtures thereof in all ratios.

13. Compounds according to Claim 1 selected from the group
| No. | Structural formula and/or name |
|---|---|
| "A1" | 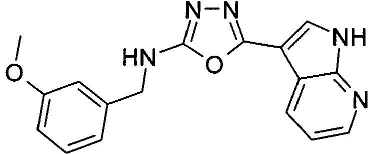 |
| "A2" | Phenyl-[5-(1 H-pyrrolo[2,3-b]pyridin-3-yl)-1,3,4-oxadiazol-2-yl]amine |
| "A3" | 5-(1 H-Pyrrolo[2,3-b]pyridin-3-yl)-1,3,4-oxadiazol-2-yl-amine |
| "A4" | 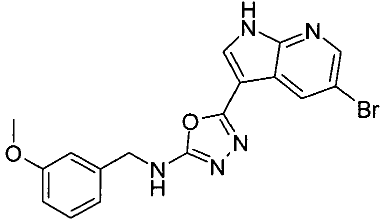 |
| "A5" | 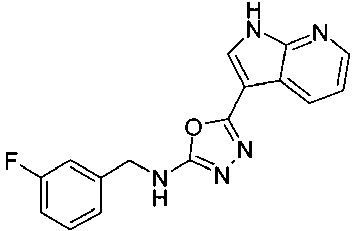 |
| "A6" | 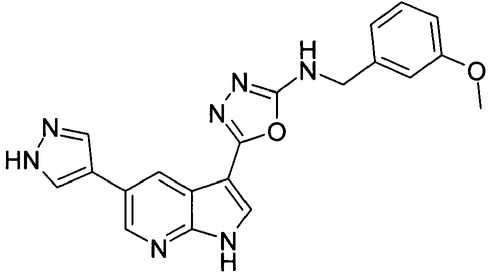 |
| "A7" | 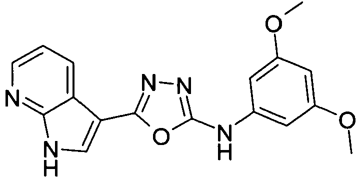 |
| "A8" | 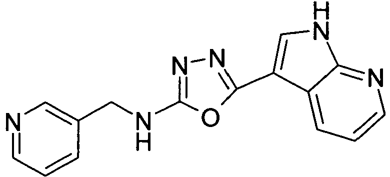 |
| "A9" | 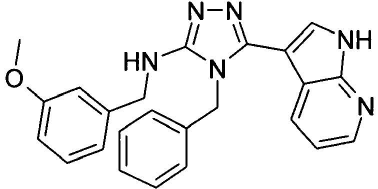 |
| "A10" | 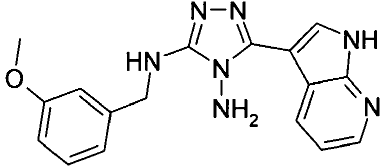 |
and pharmaceutically usable salts and stereoisomers thereof, including mixtures thereof in all ratios.

14. Process for the preparation of compounds of the formula I according to Claims 1-13 and pharmaceutically usable salts and stereoisomers thereof, **characterised in that**
a) a compound of the formula II 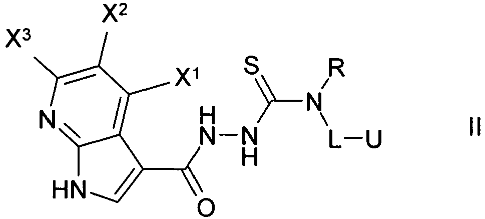 in which X¹, X², X³, R, L and U have the meanings indicated in Claim 1,
is cyclised, or
b) a compound of the formula III 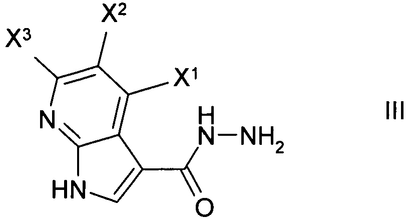 in which X¹, X², X³ have the meanings indicated in Claim 1,
is reacted with a cyanogen halide,
or
c) a compound of the formula I in which Y denotes oxygen is converted into a compound of the formula I in which Y denotes NNH₂ or N-[C(R⁷)₂]ₙAr using a hydrazine derivative or using H₂N-[C(R⁷)₂]ₙAr, and/or a base or acid of the formula I is converted into one of its salts.

15. Medicaments comprising at least one compound according to Claims 1-13 and/or pharmaceutically usable salts and stereoisomers thereof, including mixtures thereof in all ratios, and optionally excipients and/or adjuvants.

16. Use of compounds according to Claims 1-13, and pharmaceutically usable salts and stereoisomers thereof, including mixtures thereof in all ratios, for the preparation of a medicament for the treatment of diseases which are influenced by inhibition of protein kinases by the compounds according to Claims 1-13.

17. Use according to Claim 16 of compounds according to Claims 1-13, and pharmaceutically usable salts and stereoisomers thereof, including mixtures thereof in all ratios, for the preparation of a medicament for the treatment or prevention of diabetes, obesity, metabolic syndrome (dyslipidaemia), systemic and pulmonary hypertonia, cardiovascular diseases and kidney diseases, generally in any type of fibroses and inflammatory processes, cancer, tumour cells, tumour metastases, coagulopathies, neuronal excitability, glaucoma, cataract, bacterial infections and in anti-infection therapy, for increasing learning ability and attention, and for the treatment and prophylaxis of cell ageing and stress and for the treatment of tinnitus.

18. Use according to Claim 16 of compounds according to Claims 1-13, and pharmaceutically usable salts and stereoisomers thereof, including mixtures thereof in all ratios, for the preparation of a medicament for the treatment or prevention of neurodegenerative diseases, Parkinson's, tauopathies, Alzheimer's disease, corticobasal degeneration, Pick's disease, Wilson's disease, Huntington's disease, vascular dementia, acute strokes, peripheral neuropathies, retinopathy or glaucoma, manic-depressive diseases, autoimmune diseases, inflammatory and proliferative diseases, AIDS, asthma, rhinitis, Crohn's disease.

19. Use according to Claim 16 of compounds according to Claims 1-13, and pharmaceutically usable salts and stereoisomers thereof, including mixtures thereof in all ratios, for the preparation of a medicament, where the disease to be treated is a solid tumour.

20. Use according to Claim 19, where the solid tumour originates from the group of tumours of the squamous epithelium, the bladder, the stomach, the kidneys, of head and neck, the oesophagus, the cervix, the thyroid, the intestine, the liver, the brain, the prostate, the urogenital tract, the lymphatic system, the stomach, the larynx and/or the lung.

21. Use according to Claim 19, where the solid tumour originates from the group monocytic leukaemia, lung adenocarcinoma, small-cell lung carcinomas, pancreatic cancer, glioblastomas and breast carcinoma.

22. Use according to Claim 20, where the solid tumour originates from the group of lung adenocarcinoma, small-cell lung carcinomas, pancreatic cancer, glioblastomas, colon carcinoma and breast carcinoma.

23. Use according to Claim 17, where the disease to be treated is a tumour of the blood and immune system.

24. Use according to Claim 23, where the tumour originates from the group of acute myeloid leukaemia, chronic myeloid leukaemia, acute lymphatic leukaemia and/or chronic lymphatic leukaemia.

25. Medicaments comprising at least one compound according to Claims 1-13 and/or pharmaceutically usable salts and stereoisomers thereof, including mixtures thereof in all ratios, and at least one further medicament active ingredient.

26. Set (kit) consisting of separate packs of
(a) an effective amount of a compound according to Claims 1-13 and/or pharmaceutically usable salts and stereoisomers thereof, including mixtures thereof in all ratios,
and
(b) an effective amount of a further medicament active ingredient.

## Revendications

1. Composés de formule I 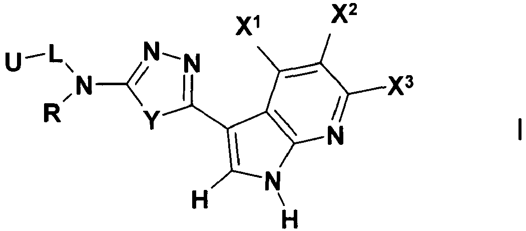 dans laquelle
L est absent ou désigne CR⁷R⁸, CR⁷R⁸CR⁹R¹⁰, CR⁷R⁸C(OR⁹)R¹⁰, NR⁷, O, NR⁶CR⁷R⁸, CR⁷R⁸NR⁹, OCR⁷R⁸, OCR⁷R⁸CR⁹R¹⁰, CR⁷R⁸O, CR⁷R⁸CR⁹R¹⁰O, NR⁶CR⁷R⁸CR⁹R¹⁰, CR⁷R⁸SO₂, NR⁷CONR⁸, NR⁷CONR⁸CR⁹R¹⁰, COCR⁷R⁸, CONR⁷, CONR⁷CR⁸R⁹, CONHNH, NR⁷CR⁸R⁹CONR¹⁰ , NR⁷CO ou NR⁷COCR⁸R⁹ ,
U désigne H, A, Ar ou Hét,
Y désigne O, NH, NNH₂ ou N-[C(R⁷)₂]ₙAr,
R désigne H ou R¹¹,
X¹, X², X³ désignent chacun, indépendamment les uns des autres, H, A, Hal, OH, OA, -[C(R⁷)₂]ₙAr, -[C(R⁷)₂]ₙHét, OAr, OHét, SH, SA, SAr, SHét, NH₂, NHA, NAA', NHAr, N(Ar)₂, NHHét, N(Hét)₂, NAAr, NAHét, SOA, SOAr, SOHét, SO₂A, SO₂Ar, SO₂Hét, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr, COHét, SO₃H, SO₂NH₂, SO₂NHAr, SO₂N(Ar)₂, SO₂NHHét ou SO₂N(Hét)₂,
R⁶, R⁷, R⁸, R⁹, R¹⁰ désignent chacun, indépendamment les uns des autres, H ou A,
R¹¹ désigne alkyle ayant 1-6 atomes de C, dans lequel 1-5 atomes de H peuvent être remplacés par F,
A, A' désignent chacun, indépendamment les uns des autres, alkyle ayant 1-10 atomes de C qui est non substitué ou mono-, diou trisubstitué par =S, =NR⁷ et/ou =O (oxygène du carbonyle) et dans lequel un, deux ou trois groupements CH₂ peuvent être remplacés par O, S, SO, S0₂, NH, NR¹¹ et/ou par des groupements -CH=CH- et/ou, en outre, 1-7 atomes de H peuvent être remplacés par F et/ou Cl, ou alkyle cyclique ayant 3-7 atomes de C,
Ar désigne phényle, naphtyle ou biphényle, chacun d'entre eux étant non substitué ou mono-, di-, tri- ou tétrasubstitué par A, Hal, OH, OA, Ar', OAr', Hét, OHét, SH, SA, SAr', SHét, NH₂, NHA, NAA', NHAr', N(Ar')₂, NHHét, N(Hét)₂, NAAr', NAHét, SOA, SOAr', SOHét, SO₂A, SO₂Ar', SO₂Hét, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', COHét, SO₃H, SO₂NH₂, SO₂NHAr', SO₂N(Ar')₂, SO₂NHHét et/ou SO₂N(Hét)₂,
Hét désigne un hétérocycle mono- ou bicyclique saturé, insaturé ou aromatique ayant 1 à 4 atomes de N, O et/ou S, qui peut être mono-, di- ou trisubstitué par A, Hal, OH, OA, Ar, OAr, Hét', OHét', SH, SA, SAr', SHét', NH₂, NHA, NAA', NHAr', N(Ar')₂, NHHét', N(Hét')₂, NAAr', NAHét', SOA, SOAr', SOHét', SO₂A, SO₂Ar', SO₂Hét', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', COHét', SO₃H, SO₂NH₂, SO₂NHAr', SO₂N(Ar')₂, SO₂NHHét' ou SO₂N(Hét')₂, =S, =NR⁷ et/ou =O (oxygène du carbonyle),
Ar' désigne phényle qui est non substitué ou mono-, di-, tri- ou tétrasubstitué par A, Hal, OH, OA, O-phényle, SH, SA, NH₂, NHA, NAA', NH-phényle, SOA, SO-phényle, SO₂A, SO₂-phényle, NO₂, CN, COOH, COOA, CONH₂, CONHA, CONA₂, NHCOA, NACOA, NHCONH₂, NHCONHA, NHCONA₂, NHSO₂A, NASO₂A, CHO, COA, CO-phényle, SO₃H, SO₂NH₂, SO₂NH-phényle et/ou SO₂N(phényl)₂,
Hét' désigne un hétérocycle mono- ou bicyclique saturé, insaturé ou aromatique ayant 1 à 4 atomes de N, O et/ou S, qui peut être mono-, di- ou trisubstitué par A, Hal, OH, OA, NH₂, NHA, NAA', SOA, SOAr', SO₂A, SO₂Ar', NO₂, CN, COOH, COOA, CONH₂, CONHA, CONA₂, NHCOA, NACOA, NHCONH₂, NHCONHA, NHCONA₂, NHSO₂A, NASO₂A, CHO, COA, COAr', SO₃H, SO₂NH₂, SO₂NHAr', SO₂N(Ar')₂, =S, =NR⁷ et/ou =O (oxygène du carbonyle),
Hal désigne F, CI, Br ou I,
n désigne 0, 1 ou 2,
et des sels pharmaceutiquement utilisables et stéréoisomères de ceux-ci, y compris des mélanges de ceux-ci en toutes proportions.

2. Composés selon la revendication 1, dans lesquels
L est absent ou désigne CR⁷R⁸,
et des sels pharmaceutiquement utilisables et stéréoisomères de ceux-ci, y compris des mélanges de ceux-ci en toutes proportions.

3. Composés selon la revendication 1 ou 2, dans lesquels
R désigne H,
et des sels pharmaceutiquement utilisables et stéréoisomères de ceux-ci, y compris des mélanges de ceux-ci en toutes proportions.

4. Composés selon l'une ou plusieurs des revendications 1-3, dans lesquels
A désigne alkyle ayant 1-10 atomes de C, dans lequel 1-7 atomes de H peuvent être remplacés par F et/ou CI,
et des sels pharmaceutiquement utilisables et stéréoisomères de ceux-ci, y compris des mélanges de ceux-ci en toutes proportions.

5. Composés selon l'une ou plusieurs des revendications 1-4, dans lesquels
Ar désigne phényle qui est non substitué ou mono-, di-, tri- ou tétrasubstitué par A, Hal, OH et/ou OA,
et des sels pharmaceutiquement utilisables et stéréoisomères de ceux-ci, y compris des mélanges de ceux-ci en toutes proportions.

6. Composés selon l'une ou plusieurs des revendications 1-5, dans lesquels
Hét désigne un hétérocycle monocyclique aromatique ayant 1 à 4 atomes de N, O et/ou S,
et des sels pharmaceutiquement utilisables et stéréoisomères de ceux-ci, y compris des mélanges de ceux-ci en toutes proportions.

7. Composés selon l'une ou plusieurs des revendications 1-6, dans lesquels
Hét désigne 2- ou 3-furyle, 2- ou 3-thiényle, 1-, 2- ou 3-pyrrolyle, 1-, 2-, 4- ou 5-imidazolyle, 1-, 3-, 4- ou 5-pyrazolyle, 2-, 4- ou 5-oxazolyle, 3-, 4- ou 5-isoxazolyle, 2-, 4- ou 5-thiazolyle, 3-, 4- ou 5-isothiazolyle, 2-, 3- ou 4-pyridyle, 2-, 4-, 5- ou 6-pyrimidinyle, 1,2,3-triazol-1-, -4- ou -5-yle, 1,2,4-triazol-1-, -3- ou 5-yle, 1- ou -5-tétrazolyle, 1,2,3-oxadiazol-4- ou -5-yle, 1,2,4-oxadiazol-3- ou -5-yle, 1,3,4-thiadiazol-2- ou -5-yle, 1,2,4-thiadiazol-3- ou -5-yle, 1,2,3-thiadiazol-4- ou -5-yle, 3- ou 4-pyridazinyle ou pyrazinyle,
et des sels pharmaceutiquement utilisables et stéréoisomères de ceux-ci, y compris des mélanges de ceux-ci en toutes proportions.

8. Composés selon l'une ou plusieurs des revendications 1-7, dans lesquels
R⁷, R⁸ désignent chacun, indépendamment les uns des autres, H ou R¹¹,
et des sels pharmaceutiquement utilisables et stéréoisomères de ceux-ci, y compris des mélanges de ceux-ci en toutes proportions.

9. Composés selon l'une ou plusieurs des revendications 1-8, dans lesquels
R⁷, R⁸ désignent chacun, indépendamment les uns des autres, H ou CH₃,
et des sels pharmaceutiquement utilisables et stéréoisomères de ceux-ci, y compris des mélanges de ceux-ci en toutes proportions.

10. Composés selon l'une ou plusieurs des revendications 1-9, dans lesquels
Hét désigne pyrrolyle, 2-, 3- ou 4-pyridyle, 2- ou 3-furyle ou 2- ou 3-thiényle,
et des sels pharmaceutiquement utilisables et stéréoisomères de ceux-ci, y compris des mélanges de ceux-ci en toutes proportions.

11. Composés selon l'une ou plusieurs des revendications 1-10, dans lesquels
X¹, X², X³ désignent chacun, indépendamment les uns des autres, H, Hal ou -[C(R⁷)₂]ₙHét,
et des sels pharmaceutiquement utilisables et stéréoisomères de ceux-ci, y compris des mélanges de ceux-ci en toutes proportions.

12. Composés selon l'une ou plusieurs des revendications 1-11, dans lesquels
L est absent ou désigne CR⁷R⁸,
U désigne H, A, Ar ou Hét,
Y désigne O, NH, NNH₂ ou N-[C(R⁷)₂]ₙAr,
R désigne H ou R¹¹,
X¹, X², X³ désignent chacun, indépendamment les uns des autres, H, Hal ou -[C(R⁷)₂]ₙHét,
R⁷, R⁸ désignent chacun, indépendamment les uns des autres, H ou R¹¹ ,
R¹¹ désigne alkyle ayant 1-6 atomes de C, dans lequel 1-5 atomes de H peuvent être remplacés par F,
A désigne alkyle ayant 1-10 atomes de C, dans lequel 1-7 atomes de H peuvent être remplacés par F et/ou Cl,
Ar désigne phényle qui est non substitué ou mono-, di-, tri- ou tétrasubstitué par A, Hal, OH et/ou OA,
Hét désigne un hétérocycle monocyclique aromatique ayant 1 à 4 atomes de N, O et/ou S,
Hal désigne F, CI, Br ou I,
n désigne 0, 1 ou 2,
et des sels pharmaceutiquement utilisables et stéréoisomères de ceux-ci, y compris des mélanges de ceux-ci en toutes proportions.

13. Composés selon la revendication 1, choisis dans le groupe constitué par
| N° | Formule structurale et/ou nom |
|---|---|
| "A1" | 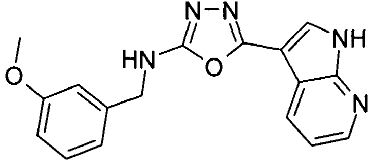 |
| "A2" | Phényl-[5-(1 H-pyrrolo[2,3-b]pyridin-3-yl)-1,3,4-oxadiazol-2-yl]amine |
| "A3" | 5-(1H-Pyrro)o[2,3-b]pyridin-3-yl)-1,3,4-oxadiazol-2-yl-amine |
| "A4" | 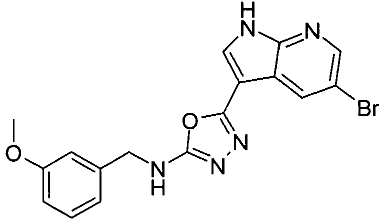 |
| "A5" | 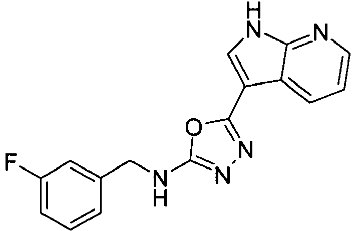 |
| "A6" | 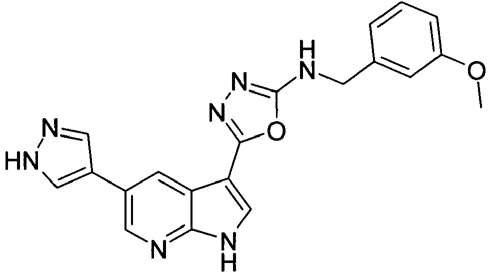 |
| "A7" | 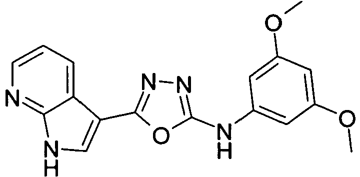 |
| "A8" | 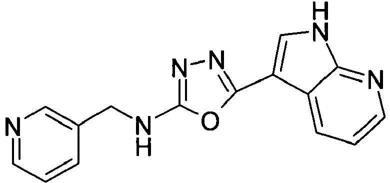 |
| "A9" | 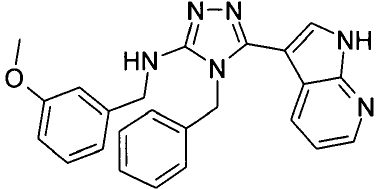 |
| "A10" | 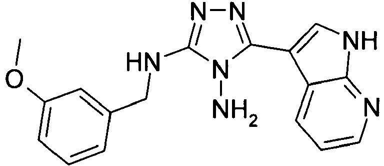 |
et des sels pharmaceutiquement utilisables et stéréoisomères de ceux-ci, y compris des mélanges de ceux-ci en toutes proportions.

14. Procédé de préparation de composés de formule I selon les revendications 1-13 et de sels pharmaceutiquement utilisables et de stéréoisomères de ceux-ci, **caractérisé en ce que**
a) un composé de formule II 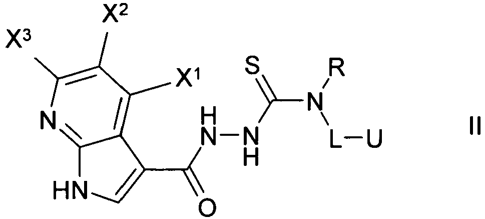 dans laquelle X¹, X², X³, R, L et U ont les significations indiquées dans la revendication 1,
est cyclisé,
ou
b) un composé de formule III 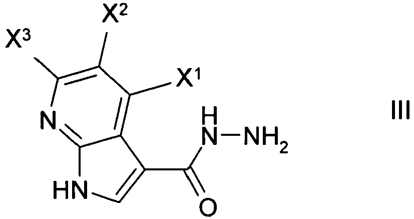 dans laquelle X¹, X², X³ ont les significations indiquées dans la revendication 1,
est réagi avec un halogénure de cyanogène,
ou
c) un composé de formule I dans laquelle Y désigne oxygène est converti en un composé de formule I dans laquelle Y désigne NNH₂ ou N-[C(R⁷)₂]ₙAr en utilisant un dérivé d'hydrazine ou en utilisant H₂N-[C(R⁷)₂]ₙAr,
et/ou une base ou un acide de formule I est converti(e) en l'un de ses sels.

15. Médicaments comprenant au moins un composé selon les revendications 1-13 et/ou des sels pharmaceutiquement utilisables et des stéréoisomères de celui-ci, y compris des mélanges de ceux-ci en toutes proportions, et éventuellement des excipients et/ou des adjuvants.

16. Utilisation de composés selon les revendications 1-13, et de sels pharmaceutiquement utilisables et de stéréoisomères de ceux-ci, y compris des mélanges de ceux-ci en toutes proportions, pour la préparation d'un médicament destiné au traitement de maladies qui sont influencées par l'inhibition de protéines kinases par les composés selon les revendications 1-13.

17. Utilisation selon la revendication 16 de composés selon les revendications 1-13, et de sels pharmaceutiquement utilisables et de stéréoisomères de ceux-ci, y compris des mélanges de ceux-ci en toutes proportions, pour la préparation d'un médicament destiné au traitement ou à la prévention du diabète, de l'obésité, du syndrome métabolique (dyslipidémie), de l'hypertonie systémique et pulmonaire, des maladies cardiovasculaires et rénales, généralement des fibroses et des processus inflammatoires de tout type, du cancer, des cellules tumorales, des métastases tumorales, des coagulopathies, de l'excitabilité neuronale, du glaucome, de la cataracte, des infections bactériennes et dans la thérapie anti-infectieuse, pour améliorer les capacités d'apprentissage et l'attention, et pour le traitement et la prophylaxie du stress et du vieillissement cellulaire et pour le traitement de l'acouphène.

18. Utilisation selon la revendication 16 de composés selon les revendications 1-13, et de sels pharmaceutiquement utilisables et de stéréoisomères de ceux-ci, y compris des mélanges de ceux-ci en toutes proportions, pour la préparation d'un médicament destiné au traitement ou de la prévention des maladies neurodégénératives, de la maladie de Parkinson, des tauopathies, de la maladie d'Alzheimer, de la dégénérescence cortico-basale, de la maladie de Pick, de la maladie de Wilson, de la chorée de Huntington, de la démence vasculaire, des accidents vasculaires cérébraux aigus, des neuropathies périphériques, de la rétinopathie ou du glaucome, des maladies maniaco-dépressives, des maladies auto-immunes, des maladies inflammatoires et prolifératives, du SIDA, de l'asthme, de la rhinite, de la maladie de Crohn.

19. Utilisation selon la revendication 16 de composés selon les revendications 1-13, et de sels pharmaceutiquement utilisables et de stéréoisomères de ceux-ci, y compris des mélanges de ceux-ci en toutes proportions, pour la préparation d'un médicament, où la maladie à traiter est une tumeur solide.

20. Utilisation selon la revendication 19, dans laquelle la tumeur solide provient du groupe constitué par les tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, du larynx et/ou du poumon.

21. Utilisation selon la revendication 19, dans laquelle la tumeur solide provient du groupe constitué par la leucémie monocytique, l'adénocarcinome du poumon, les carcinomes du poumon à petites cellules, le cancer du pancréas, les glioblastomes et le carcinome du sein.

22. Utilisation selon la revendication 20, dans laquelle la tumeur solide provient du groupe constitué par l'adénocarcinome du poumon, les carcinomes du poumon à petites cellules, le cancer du pancréas, les glioblastomes, le carcinome du côlon et le carcinome du sein.

23. Utilisation selon la revendication 17, dans laquelle la maladie à traiter est une tumeur du système sanguin et immunitaire.

24. Utilisation selon la revendication 23, dans laquelle la tumeur provient du groupe constitué par la leucémie myéloïde aiguë, la leucémie myéloïde chronique, la leucémie lymphatique aiguë et/ou la leucémie lymphatique chronique.

25. Médicaments comprenant au moins un composé selon les revendications 1-13 et/ou des sels pharmaceutiquement utilisables et des stéréoisomères de celui-ci, y compris des mélanges de ceux-ci en toutes proportions, et au moins un autre ingrédient actif médicamenteux.

26. Ensemble (kit) constitué de conditionnements séparés
(a) d'une quantité efficace d'un composé selon les revendications 1-13 et/ou de sels pharmaceutiquement utilisables et de stéréoisomères de celui-ci, y compris des mélanges de ceux-ci en toutes proportions,
et
(b) d'une quantité efficace d'un autre ingrédient actif médicamenteux.
